# EUROPEAN PATENT SPECIFICATION

(11) **EP 1 280 760 B1**
(45) Date of publication and mention of the grant of the patent: **28.04.2004**
(21) Application number: 01929156.6
(22) Date of filing: 11.05.2001
(51) Int. Cl.: C07C 229/50, A61K 31/196, A61P 25/00, C07C 229/46

(54) **NOVEL SPIRO[2.4]HEPTANE AMINO CARBOXY COMPOUNDS AND DERIVATIVES THEREOF**
SPIRO[2.4]HEPTANAMINOCARBONSÄURE UND IHRE DERIVATE
NOUVEAUX COMPOSES SPIRO[2.4]HEPTANE AMINO CARBOXY ET DERIVES ASSOCIES

(30) Priority: 11.05.2000 CA 2308536
(43) Date of publication of application: 05.02.2003
(73) Proprietor: Curry, Kenneth, Vancouver, British Columbia V6M 2V3 (CA)
(72) Inventor: Curry, Kenneth, Vancouver, British Columbia V6M 2V3 (CA)
(74) Representative: Bassil, Nicholas Charles
(86) International application number: PCT/CA2001/000650
(87) International publication number: WO 2001/085669

(56) References cited:
- DE-A- 2 130 084
- US-A- 5 500 420
- CURRY, KENNETH ET AL: "Synthesis, resolution, and absolute configuration of the isomers of the neuronal excitant 1-amino-1,3-cyclopentanedicarboxylic acid" J. MED. CHEM. (1988), 31(4), 864-7 , XP001024209
- GOTI, ANDREA ET AL: "Thermal Rearrangement of Nitrone and Nitrile Oxide Cycloadducts to Bicyclopropylidene. Synthesis of 3-Spirocyclopropane-4-pyridone and Furo[2,3-c]pyridine Derivatives" J. ORG. CHEM. (1996), 61(5), 1665-72 , XP001028020

## Description

This invention pertains to therapeutically active novel spiro[2.4]heptane amino carboxy compounds and derivatives thereof, a method for preparing the same, pharmaceutical compositions comprising the compounds and a method of treating diseases of the Central Nervous System (CNS) therewith.

### BACKGROUND OF THE INVENTION

The acidic amino acid L-glutamate is recognized as the major excitatory neurotransmitter in the CNS. The receptors that respond to L-glutamate are called excitatory amino acid receptors. The excitatory amino acid receptors are thus of great physiological importance, playing a role in a variety of physiological processes, such as long-term potentiation (learning and memory), the development of synaptic plasticity, motor control, respiratory and cardiovascular regulation, and sensory perception.

Excitatory amino acid receptors are classified into two general types and both are activated by L-glutamate and its analogs. Receptors activated by L-glutamate that are directly coupled to the opening of cation channels in the cell membrane of the neurons are termed "ionotropic." This type of receptor has been subdivided into at least three subtypes, which are defined by the depolarizing actions of the selective agonists N-Methyl-D-aspartate (NMDA), α-Amino-3-hydroxy-5-methylisoxazole-4-propionic acid (AMPA), and Kainic acid (KA).

The second general type of receptor is the G-protein or second messenger-linked "metabotropic" excitatory amino acid receptor. This second type is coupled to multiple second messenger systems that lead to enhanced phosphoinositide hydrolysis, activation of phospholipase D, increases or decreases in cAMP formation, and changes in ion channel function (Schoepp and Conn, *Trends in Pharmacological Science,* 14:13, 1993). Both types of receptors appear not only to mediate normal synaptic transmission along excitatory pathways but also to participate in the modification of synaptic connections during development and throughout life.

So far eight different clones of the G-protein-coupled mGluRs have been identified (Knopfel *et al*., 1995, *J. Med. Chem*., 38, 1417-1426). These receptors function to modulate the presynaptic release of L-glutamate, and the postsynaptic sensitivity of the neuronal cell to L-glutamate excitation. Based on pharmacology, sequence homology and the signal transduction pathway that they activate, the mGluRs have been subclassified into three groups. The mGluR1 and mGluR5 receptors form group I. They are coupled to hydrolysis of phosphatidylinositol (PI) and are selectively activated by (*RS*)-3,5-dihydroxyphenylglycine (Brabet *et al*., *Neuropharmacology,* 34, 895-903, 1995). Group II comprises mGluR2 and mGluR3 receptors. They are negatively coupled to adenylate cyclase and are selectively activated by (2*S*,1'*R*,2'*R*,3'*R*)-2-(2,3-dicarboxycyclopropyl)glycine (DCG-IV; Hayashi *et al*., *Nature*, 366, 687-690, 1993). Finally, the mGluR4, mGluR6, mGluR7 and mGluR8 receptors belong to group III. They are also negatively coupled to adenylate cyclase and are selectively activated by (*S*)-2-amino-4-phosphonobutyric acid (L-AP4; Knopfel *et al*., 1995, *J. Med Chem*., 38, 1417-1426).

Agonists and antagonists of these receptors are believed useful for the treatment of acute and chronic neurodegenerative conditions, and as antipsychotic, anticonvulsant, analgesic, anxiolytic, antidepressant, and anti-emetic agents. Antagonists and agonists of neural receptors are classified as selective for a particular receptor or receptor subtype, or as non-selective. Antagonists may also be classified as competitive or non-competitive. While competitive and non-competitive antagonists act on the receptors in a different manner to produce similar results, selectivity is based upon the observations that some antagonists exhibit high levels of activity at a single receptor type, and little or no activity at other receptors.

In the case of receptor-specific diseases and conditions, the selective agonists and antagonists are of the most value.

Compounds such as L-glutamate, quisqualate and ibotenate are known to act as non-selective agonists on the mGluRs, while selective ionotropic glutamate receptor agonists such as NMDA, AMPA and kainate have little effect on these receptors. Recently a few compounds without activity at the ionotropic glutamate receptors but with activity at the metabotropic receptors have been identified. These include *trans*-ACPD (*trans* (1*S*,3*R*-1-aminocyclopentane-1,3-dicarboxylic acid), the partial agonist L-AP3 (L-2-amino-3-phosphonopropionic acid; Palmer, E., Monaghan, D. T. and Cotman, C. W. *Eur. J. Pharmacol*. 166, 585-587, 1989; Desai, M. A. and Conn, P. J. *Neuroscience Lett*. 109, 157-162, 1990; Schoepp, D. D. *et al*., *J. Neurochemistry*. 56, 1789-1796, 1991; Schoepp D. D. and Johnson B. G. *J. Neurochemistry* 53, 1865-1613, 1989), L-AP4 (L-2-amino-4-phosphonobutyrate) which is an agonist at the mGluR4 receptor (Thomsen C. *et al*., *Eur. J. Pharmacol*. 227, 361-362, 1992) and some of the isomers of CCG (2-(carboxycyclopropyl)glycines) especially L-CCG-I and L-CCG-II (Hayashi, Y. *et al*., *Br. J. Pharmacol*. 107, 539-543, 1992).

Very few selective antagonists at the mGluRs have been reported. However some phenylglycine derivatives, *S*-4CPG (*S*-4-carboxyphenylglycine), *S*-4C3HPG (*S*-4-carboxy -3-hydroxyphenylglycine) and *S*-MCPG (*S*-α-methyl-4-carboxyphenylglycine) have been reported to antagonize *trans*-ACPD- stimulated phosphoinositide hydrolysis and thus possibly act as antagonists at mGluR1 and mGluR5 subtypes (Thomsen, C. and Suzdak, P, *Eur. J. Pharmacol*. 245, 299, 1993).

Research directed towards mGluRs is beginning to show that mGluRs may be implicated in a number of normal as well as pathological mechanisms in the brain and spinal cord. For example, activation of these receptors on neurons can influence levels of alertness, attention and cognition; protect nerve cells from excitotoxic damage resulting from ischemia, hypoglycemia and anoxia; modulate the level of neuronal excitation; influence central mechanisms involved in controlling movement; reduce sensitivity to pain; reduce levels of anxiety.

The use of compounds active at the mGluRs for the treatment of epilepsy is corroborated by investigations of the influence of *trans*-ACPD on the formation of convulsions (Sacaan and Schoepp, *Neuroscience Lett.* 139, 77, 1992) and that phosphoinositide hydrolysis mediated via mGluR is increased after kindling experiments in rats (Akiyama *et al. Brain Res. 569*, 71, 1992). *Trans-ACPD* has been shown to increase release of dopamine in the rat brain, which indicates that compounds acting on the mGluRs might be usable for the treatment of Parkinson's disease and Huntington's Chorea (Sacaan *et al*., *J. Neurochemistry* 59, 245, 1992).

*Trans*-ACPD has also been shown to be a neuroprotective agent in a medial cerebral artery occlusion (MCAO) model in mice (Chiamulera *et al. Eur. J. Pharmacol*. 215, 353, 1992), and it has been shown to inhibit NMDA-induced neurotoxicity in nerve cell cultures (Koh *et al*., *Proc. Natl. Acad Sci. USA* 88, 9431, 1991). The mGluR-active compounds are also implicated in the treatment of pain. This is proved by the fact that antagonists at the metabotropic glutamate receptors antagonizes sensory synaptic response to noxious stimuli of thalamic neurons (Eaton, S. A. *et al*., *Eur. J. Neuroscience*, 5, 186, 1993).

The use of compounds active at the mGluRs for treatment of neurological diseases such as senile dementia have also been indicated by the findings of Zheng and Gallagher (*Neuron* 9, 163, 1992) and Bashir *et al.* (*Nature* 363, 347, 1993) who demonstrated that activation of mGluRs is necessary for the induction of long term potentiation (LTP) in nerve cells (septal nucleus, hippocampus) and the finding that long term depression is induced after activation of metabotropic glutamate receptors in cerebellar granule cells (Linden *et al. Neuron* 7, 81, 1991).

Thus, compounds that demonstrate either activating or inhibiting activity at mGluRs have therapeutic potential for the treatment of neurological disorders. These compounds have application as new drugs to treat both acute and chronic neurological disorders, such as stroke and head injuries; epilepsy; movement disorders associated with Parkinson's disease and Huntington's chorea; pain; anxiety; AIDS dementia; and Alzheimer's disease. Since the GluRs can influence levels of alertness, attention and cognition; protect nerve cells from excitotoxic damage resulting from ischemia, hypoglycemia and anoxia; modulate the level of neuronal excitation; influence central mechanisms involved in controlling movement; reduce sensitivity to pain; and reduce levels of anxiety, these compounds can also be used to influence these situations and also find use in learning and memory deficiencies such as senile dementia. mGluRs may also be involved in addictive behavior, alcoholism, drug addiction, sensitization and drug withdrawal *(Science,* 280:2045, 1998), so compounds acting at mGluRs might also be used to treat these disorders.

The current pharmaceutical options for treating neurological disorders tend to be very general and non-specific in their actions in that, although they may reduce the clinical symptoms associated with a specific neurological disorder, they may also negatively impact normal function of the central nervous system of patients. Thus new cellular targets and drugs that are more specific in their actions require identification and development and a need remains for chemical compounds that demonstrate specific binding characteristics towards mGluRs.

### SUMMARY OF THE INVENTION

It is an object of this invention to provide novel spiro[2.4]heptane amino carboxy compounds and derivatives thereof, that demonstrate activity at the various metabotropic glutamate receptors. In one aspect of the present invention there is provided a compound of Formula (I) and stereoisomers thereof or pharmaceutically acceptable salts or hydrates thereof, 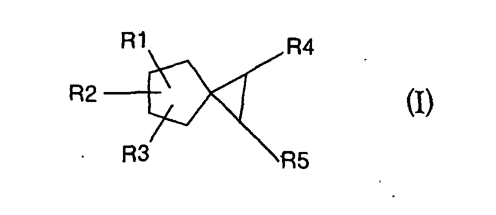 wherein:
**R1** is (CH₂)ₙ(CH)ₘXY, where: n is 0-3, m is 0 or 1, X is CO₂H and Y is NH₂, with the proviso that, when m = 0, then n = 0 and the groups X and Y are directly attached to the ring,
**R2** and **R3** can be same or different and selected from the group comprising H, halo, alkyl, cycloalkyl, aryl or heterocycle, or when **R2** and **R3** are present on adjacent carbon atoms and taken together then **R2** and **R3** can form a cycloalkyl (3-6 carbon atoms), heterocycle, or an aromatic ring or heteroaromatic ring,
**R4** is selected from the group comprising H, halo, alkyl, cycloalkyl, aryl, or heterocyclic,
**R5** is selected from the group comprising of carboxyl, phosphono, phosphino, sulfono, sulfino, borono, tetrazol, isoxazol, -CH₂-carboxyl, -CH₂-phosphono, -CH₂-phosphino, -CH₂-sulfono, -CH₂-sulfino, -CH₂-borono, -CH₂-tetrazol, -CH₂-isoxazol, and higher homologues thereof.

In accordance with further aspect of the present invention, there is provided a process for the preparation of a compound of Formula I, 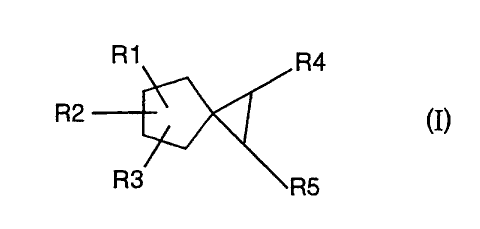 stereoisomers thereof, or pharmaceutically acceptable salts or hydrates thereof, wherein:
**R1** is (CH₂)ₙ(CH)ₘXY, where: n is 0-3, m is 0 or 1, X is CO₂H and Y is NH₂, with the proviso that, when m = 0, then n = 0 and the groups X and Y are directly attached to the ring,
**R2** and **R3** can be same or different and selected from the group comprising H, halo, alkyl, cycloalkyl, aryl or heterocycle, or when R2 and R3 are present on adjacent carbon atoms and taken together then R2 and R3 can form a cycloalkyl (3-6 carbon atoms), heterocycle, an aromatic ring or heteroaromatic ring,
**R4** is selected from the group comprising H, halo, alkyl, cycloalkyl, aryl or heterocycle,
**R5** is selected from the group comprising carboxyl, phosphono, phosphino, sulfono, sulfino, borono, tetrazol, isoxazol, -CH₂-carboxyl, -CH₂-phosphono, -CH₂-phosphino, -CH₂-sulfono, -CH₂-sulfino, -CH₂-borono, -CH₂-tetrazol, -CH₂-isoxazol, and higher homologues thereof, which comprises:
   (a) hydrolyzing a compound of formula II: 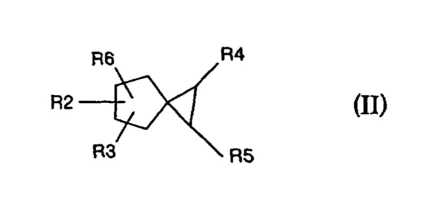 wherein: **R2, R3, R4, R5** are as defined above, and **R6** is: 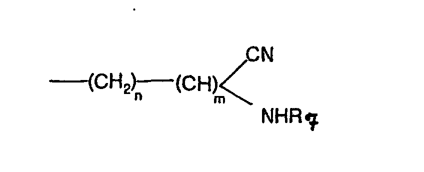 wherein:
      n is 0-3, m is 0 or 1, with the proviso that, when m = 0, then n= 0 and the groups CN and NHR7 are directly attached to the ring, **R7** represents a hydrogen atom or an acyl group. Preferred values for **R7** are hydrogen and (C₁-C₂) alkanoyl groups, such as acetyl,
   (b) hydrolyzing a compound of formula III: 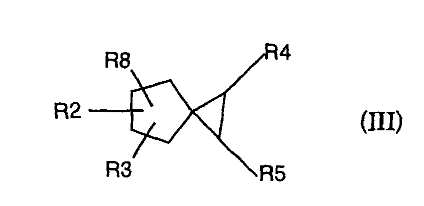 wherein: **R2, R3, R4, R5** are as defined above, and **R8** is: 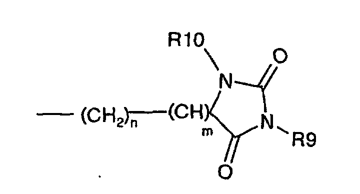 wherein: n is 0-3, m is 0 or 1, with the proviso that, when m = 0, then n= 0 and the cyclic group containing R9 and R10 is directly attached to the 5-membered ring, **R9** and **R10** each independently represent a hydrogen atom, a (C₂-C₆) alkanoyl group, a (C₁-C₄) alkyl group, a (C₂-C₄) alkenyl group or a phenyl (C₁-C₄) alkyl group in which the phenyl is unsubstituted or substituted by halogen, (C₁-C₄) alkyl or (C₁-C₄) alkoxy, or a salt thereof; or
   (c) deprotecting a compound of formula IV: 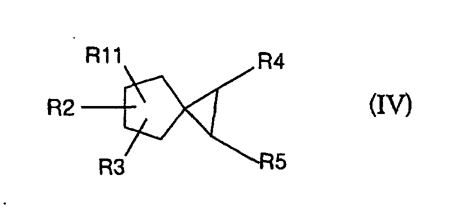 wherein: **R2, R3, R4, R5** are as defined above, and **R11** is: 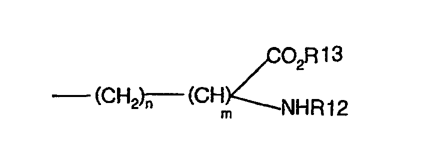 wherein:
      n is 0-3, m is 0 or 1, with the proviso that, when m = 0, then n = 0 and the groups CO₂R13 and NHR12 are directly attached to the ring, **R13** represents a hydrogen atom or a carboxyl protecting group, or a salt thereof, and **R12** represents a hydrogen atom or a nitrogen protecting group; whereafter, if necessary and/or desired the following steps are carried out:
      (i) resolving the compound of Formula I;
      (ii) converting the compound of Formula I into a non-toxic metabolically-labile ester or amide thereof; and/or;
      (iii) converting the compound of Formula I or a non-toxic metabolically-labile ester or amide thereof into a pharmaceutically acceptable salt thereof.

According to further aspect of the invention there is provided a compound of formula: 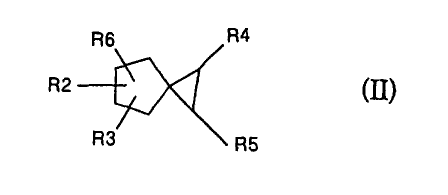 wherein: **R2, R3, R4, R5** are as defined above, and **R6** is: 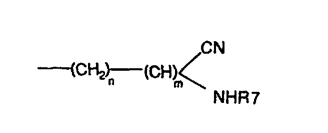 wherein:
n is 0-3, m is 0 or 1, with the proviso that, when m = 0, then n= 0 and the groups CN and NHR7 are directly attached to the ring, **R7** represents a hydrogen atom or an acyl group. Preferred values for **R7** are hydrogen and (C₁-C₂) alkanoyl groups, such as acetyl,

According to further aspect of the invention there is provided a compound of formula: 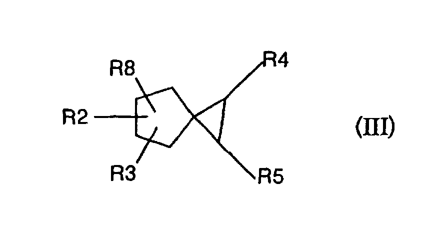 wherein: **R2, R3, R4, R5** are as defined above, and **R8** is: 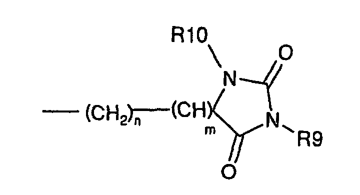 wherein: n is 0-3, m is 0 or 1, with the proviso that, when m = 0, then n= 0 and the cyclic group containing **R9** and **R10** is directly attached to the five membered ring, **R9** and **R10** each independently represent a hydrogen atom, a (C₂-C₆) alkanoyl group, a (C₁-C₄) alkyl group, a (C₂-C₄) alkenyl group or a phenyl (C₁-C₄) alkyl group in which the phenyl is unsubstituted or substituted by halogen, (C₁-C₄) alkyl or (C₁-C₄) alkoxy, or a salt thereof.

According to further aspect of the invention there is provided a compound of formula: 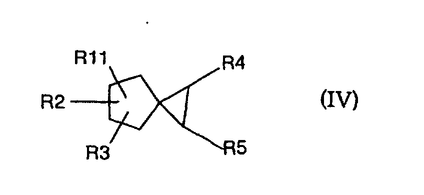 wherein: **R2, R3, R4, R5** are as defined above, and **R11** is: 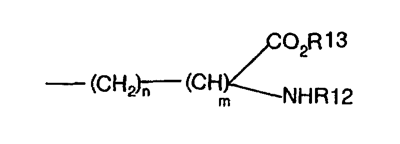 wherein:
n is 0-3, m is 0 or 1, with the proviso that, when m = 0, then n = 0 and the groups CO₂R13 and NHR12 are directly attached to the ring, **R13** represents a hydrogen atom or a carboxyl protecting group, or a salt thereof, and **R12** represents a hydrogen atom or a nitrogen protecting group.

### DETAILED DESCRIPTION OF THE INVENTION

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "mL" means milliliter or milliliters; "M" refers to molar or molarity; "MS" refers to mass spectrometry; "IR" refers to infrared spectroscopy; and "NMR" refers to nuclear magnetic resonance spectroscopy.

Alkyl: refers to a saturated straight chain, or branched hydrocarbon group containing 1-10 carbons. Typical alkyl groups include, but not limited to methyl, ethyl, propyl, isopropyl, butyl, isobutyl, *t*-butyl, pentyl, isopentyl and hexyl.

Cycloalkyl: refers to a mono- or polycyclic hydrocarbon group that contains 3 to 15 carbons and may optionally contain one or two double bonds. Typical cycloalkyl groups include, but not limited to cyclopropyl, cyclobutyl, cyclopentyl and cyclohexyl.

Heterocyclic or heterocycle: refers to a stable mono- or polycyclic compound which may optionally contain one or two double bonds or may optionally contain one or more aromatic rings. Each heterocycle consists of carbon atoms and from one to four heteroatoms independently selected from a group including nitrogen, oxygen and sulfur and include any oxidized form of nitrogen and sulfur and quatemized form of any basic nitrogen. Typical heterocyclic groups include, but not limited to pyrimidinyl, tetrahydroquinolyl, tetrahydroisoquinonlinyl, purinyl, pyrimidyl, indolinyl, benzimidazolyl, imidazolyl, imidazolinoyl, imidazolidinyl, quinolyl, isoquinolyl, indolyl, pyridyl, pyrrolyl, pyrrolinyl, pyrazolyl, pyrazinyl, quinoxolyl, piperidinyl, piperazinyl, morpholinyl, thiamorpholinyl, furyl, thienyl, triazolyl and thiazolyl. Further heterocycles are described in A. R. Katrizky and C. W. Rees, eds., *Comprehensive Heterocyclic Chemistry: The Structure, Reactions, Synthesis and Use of Heterocyclic Compounds,* Vol. 1-8, Pergamon Press, N.Y. (1984).

Aryl: refers to a mono- or polycyclic group which contains 6, 10, 12, or 14 carbons In which at least one ring is aromatic. Typical aryl groups include, but not limited to, phenyl, naphthyl, anthracyl and azulenyl.

Heteroaromatic: refers to a mono- or polycyclic group which contains 1 to 15 carbon atoms and from 1 to 4 heteroatoms, each of which is selected independently from a group including sulfur, nitrogen and oxygen, and which additionally contains 1 to 3, five or six membered rings, at least one of which is aromatic.

As would be understood by the skilled artisan throughout the synthesis of the compounds of Formula I, it may be necessary to employ an amino-protecting group or a carboxy-protecting group in order to reversibly preserve a reactively susceptible amino or carboxy functionality while reacting other functional groups on the compound.

Examples of such amino-protecting groups include formyl, trityl, phthalimido, trichloroacetyl, chloroacetyl, bromoacetyl, iodoacetyl, and urethane-type blocking groups such as benzyloxycarbonyl, 4-phenylbenzyloxycarbonyl, 2-methylbenzyloxycarbonyl, 4-methoxybenzyloxycarbonyl, 4-fluorobenzyloxycarbonyl, 4-chlorobenzyloxycarbonyl, 3-chlorobenzyloxycarbonyl, 2-chlorobenzyloxycarbonyl, 2,4-dichlorobenzyloxycarbonyl, 4-bromobenzyloxycarbonyl, 3-bromobenzyloxycarbonyl, 4-nitrobenzyloxycarbonyl, 4-cyanobenzyloxycarbonyl, t-butoxycarbonyl, 2-(4-xenyl)-isopropoxycarbonyl, 1,1-diphenyleth-1-yloxycarbonyl, 1,1-diphenylprop-1-yloxycarbonyl, 2-phenylprop-2-yloxycarbonyl, 2-(*p*-toluyl)-prop-2-yloxycarbonyl, cyclopentanyloxy-carbonyl, 1-methylcyclopentanyloxycarbonyl, cyclohexanyloxycarbonyl, 1-methylcyclohexanyloxycarbonyl, 2-methylcyclohexanyloxycarbonyl, 2-(4-toluylsulfono)-ethoxycarbonyl, 2-(methylsulfono)ethoxycarbonyl, 2-(triphenylphosphino)-ethoxycarbonyl, fluorenylmethoxycarbonyl ("FMOC"), 2-(trimethylsilyl)ethoxycarbonyl, allyloxycarbonyl, 1-(trimethylsilylmethyl)prop-1-enyloxycarbonyl, 5-benzisoxalylmethoxycarbonyl, 4-acetoxybenzyloxycarbonyl, 2,2,2-trichloroethoxycarbonyl, 2-ethynyl-2-propoxycarbonyl, cyclopropylmethoxycarbonyl, 4-(decycloxy)benzyloxycarbonyl, isobornyloxycarbonyl, 1-piperidyloxycarbonlyl; benzoylmethylsulfono group, 2-nitrophenylsulfenyl, diphenylphosphine oxide and like amino-protecting groups. The species of amino-protecting group employed is not critical so long as the derivatized amino group is stable to the condition of subsequent reaction(s) on other positions of the intermediate molecule and can be selectively removed at the appropriate point without disrupting the remainder of the molecule including any other amino-protecting group(s). Preferred amino-protecting groups are *t*-butoxycarbonyl (*t*-Boc), allyloxycarbonyl and benzyloxycarbonyl (CbZ). Further examples of these groups are found in E. Haslam in *Protective Groups in Organic Synthesis;* McOmie, J. G. W., Ed. 1973, at Chapter 2; and Greene, T.W. and Wuts, P. G. M., *Protective Groups in Organic Synthesis,* Second edition; Wiley-Interscience: 1991; Chapter 7.

Examples of carboxyl-protecting groups include methyl, *p*-nitrobenzyl, *p*-methylbenzyl, *p*-methoxybenzyl, 3,4-dimethoxybenzyl, 2,4-dimethoxybenzyl, 2,4,6-trimethoxybenzyl, 2,4,6-trimethylbenzyl, pentamethylbenzyl, 3,4-methylenedioxybenzyl, benzhydryl, 4,4'-dimethoxybenzhydryl, 2,2',4,4'-tetramethoxybenzhydryl, *t*-butyl, *t*-amyl, trityl, 4-methoxytrityl, 4,4'-dimethoxytrityl, 4,4',4"-trimethoxytrityl, 2-phenylprop-2-yl, trimethylsilyl, t-butyldimethylsilyl, phenacyl, 2,2,2-trichloroethyl, β-(di(n-butyl)methylsilyl)ethyl, *p*-toluenesulfonoethyl, 4-nitrobenzylsulfonoethyl, allyl, cinnamyl, 1-(trimethylsilylmethyl)prop-1-en-3-yl. Preferred carboxyl-protecting groups are allyl, benzyl and t-butyl. Further examples of these groups are found in E. Haslam, supra, at Chapter 5; and T. W. Greene and P. G. M. Wuts, supra, at Chapter 5.

The present invention provides a compound of the formula (I), stereoisomers thereof, or pharmaceutically acceptable salts or hydrates thereof: 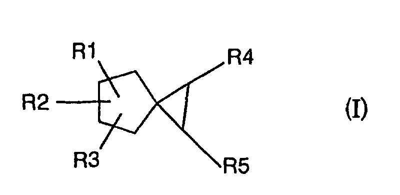 wherein:
**R1** is (CH₂)ₙ(CH)ₘXY, where: n is 0-3, m is 0 or 1, X is CO₂H and Y is NH₂, with the proviso that, when m = 0, then n = 0 and the groups X and Y are directly attached to the ring,
**R2** and **R3** can be same or different and selected from the group comprising H, halo, alkyl, cycloalkyl, aryl or heterocycle, or when **R2** and **R3** are present on adjacent carbon atoms and taken together then **R2** and **R3** can form a cycloalkyl (3-6 carbon atoms), heterocycle, or an aromatic ring or heteroaromatic ring,
**R4** is selected from the group comprising H, halo, alkyl, cycloalkyl, aryl or heterocycle,
**R5** is selected from the group comprising of carboxyl, phosphono, phosphino, sulfono, sulfino, borono, tetrazol, isoxazol, -CH₂-carboxyl, -CH₂-phosphono, -CH₂-phosphino, -CH₂-sulfono, -CH₂-sulfino, -CH₂-borono, -CH₂-tetrazol, -CH₂-isoxazol, and higher homologues thereof;

Compounds of the present invention include, but are not limited to the following examples:

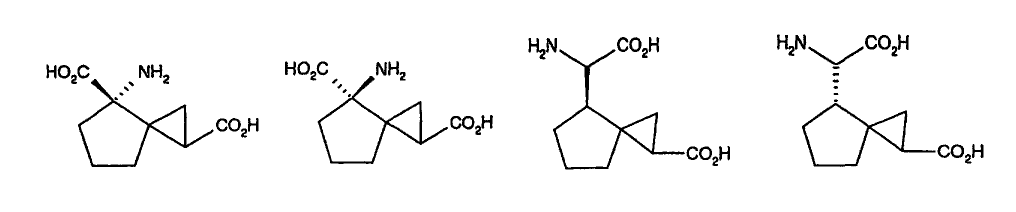

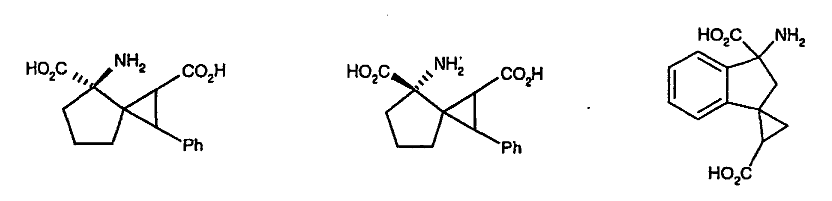

The present invention includes the pharmaceutically acceptable salts of the compounds defined by Formula I. A compound of this invention can possess a sufficiently acidic, a sufficiently basic, or both functional groups, and accordingly react with any of a number of organic and inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts.

Acids commonly employed to form acid addition salts are inorganic acids such as hydrochloric acid, hydrobromic acid, hydriodic acid, sulfuric acid, phosphoric acid, and organic acids such as *p*-toluenesulfonic acid, methanesulfonic acid, oxalic acid, *p*-bromophenylsulfonic acid, carbonic acid, succinic acid, citric acid, benzoic acid and acetic acid. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprylate, acrylate, formate, hydrochloride, dihydrochloride, isobutyrate, caproate, heptanoate, propiolate, oxalate malonate, succinate, suberate, sebacate, fumarate, maleate, butyne-1,4-dioate, hexyne-1,6-dioate, benzoate, chlorobenzoate, methylbenzoate, hydroxybenzoate, methoxybenzoate, phthalate, xylenesulfonate, phenylacetate, phenylpropionate, phenylbutyrate, citrate, lactate, gamma-hydroxybutyrate, glycolate, tartrate, methanesulfonate, propanesulfonate, naphthalene-1-sulfonate, napththalene-2-sulfonate and mandelate. Preferred pharmaceutically acceptable acid addition salts are those formed with mineral acids such as hydrochloric acid and hydrobromic acid, and those formed with organic acids such as maleic acid and methanesulfonic acid.

Salts of amine groups may also comprise quartemary ammonium salts in which the amino nitrogen carries a suitable organic group such as an alkyl, alkenyl, alkynyl, or aralkyl moiety.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide and calcium carbonate. The potassium and sodium salt forms are particularly preferred.

It should be recognized that the particular counterion forming a part of any salt of this invention is usually not of a critical nature, so long as the salt as a whole is pharmacologically acceptable and as long as the counterion does not contribute undesired qualities to the salt as a whole. This invention further encompasses the pharmaceutically acceptable solvates of the compounds of Formula I. Many of the Formula I compounds can combine with solvents such as water, methanol, ethanol and acetonitrile to form pharmaceutically acceptable solvates such as the corresponding hydrate, methanolate, ethanolate and acetonitrilate.

The compounds of the present invention have multiple asymmetric (chiral) centers. As a consequence of these chiral centers, the compounds of the present invention occur as racemates, mixtures of enantiomers and as individual enantiomers, as well as diastereomers and mixtures of diastereomers. All asymmetric forms, individual isomers and combinations thereof, are within the scope of the present invention.

The prefixes "*R*" and "*S*" are used herein as commonly used in organic chemistry to denote the absolute configuration of a chiral center, according to the Cahn-Ingold-Prelog system. The stereochemical descriptor *R* (*rectus*) refers to that configuration of a chiral center with a clockwise relationship of groups tracing the path from highest to second-lowest priorities when viewed from the side opposite to that of the lowest priority group. The stereochemical desciptor *S* (*sinister*) refers to that configuration of a chiral center with a counterclockwise relationship of groups tracing the path from highest to second-lowest priority when viewed from the side opposite to the lowest priority group. The priority of groups is decided using sequence rules as described by Cahn *et al*., *Angew. Chem*., 78, 413-447, 1966 and Prelog, V. and Helmchen, G.; *Angew. Chem. Int. Ed. Eng*., 21, 567-583, 1982).

In addition to the *R,S* system used to designate the absolute configuration of a chiral center, the older D-L system is also used in this document to denote relative configuration, especially with reference to amino acids and amino acid derivatives. In this system a Fischer projection of the compound is oriented so that carbon-1 of the parent chain is at the top. The prefix "D" is used to represent the relative configuration of the isomer in which the functional (determining) group is on the right side of the carbon atom at the chiral center and "L", that of the isomer in which it is on the left.

As would be expected, the stereochemistry of the Formula I compounds is critical to their potency as agonists or antagonists. The relative stereochemistry is established early during synthesis, which avoids subsequent stereoisomer separation problems later in the process. Further manipulation of the molecules then employs stereospecific procedures so as to maintain the preferred chirality. The preferred methods of this invention are the methods employing those preferred compounds.

Non-toxic metabolically-labile ester and amide of compounds of Formula I are ester or amide derivatives of compound of Formula I that are hydrolyzed in vivo to afford said compounds of Formula I and a pharmaceutically acceptable alcohol or amine. Examples of metabolically-labile esters include esters formed with (C₁-C₆) alkanols in which the alkanol moiety may be optionally substituted by a (C₁-C₈) alkoxy group, for example methanol, ethanol, propanol and methoxyethanol. Examples of metabolically-labile amides include amides formed with amines such as methylamine.

### Preparation of Compounds of Formula (I)

According to another aspect, the present invention provides a process for the preparation of a compound of Formula I, or a pharmaceutically acceptable metabolically-labile ester or amide thereof, or a pharmaceutically acceptable salt thereof, which comprises:
(a) hydrolyzing a compound of formula: II 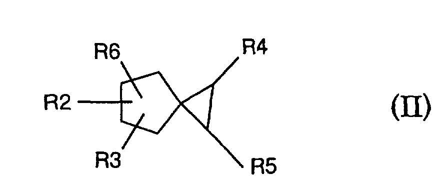 wherein: **R2, R3, R4, R5** are as defined above, and **R6** is: 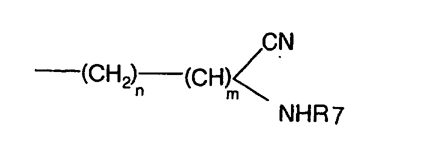 wherein:
   n is 0-3, m is 0 or 1, with the proviso that, when m = 0, then n= 0 and the groups CN and NHR7 are directly attached to the ring, **R7** represents a hydrogen atom or an acyl group. Preferred values for **R7** are hydrogen and (C₁-C₂) alkanoyl groups, such as acetyl,
(b) hydrolyzing a compound of formula III 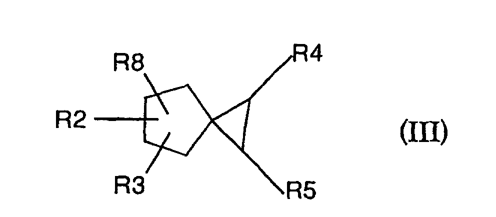 wherein: **R2, R3, R4, R5** are as defined above, and **R8** is: 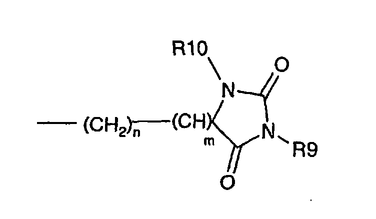 wherein: n is 0-3, m is 0 or 1, with the proviso that, when m = 0, then n= 0 and the cyclic group containing R9 and R10 is directly attached to five membered ring, **R9** and **R10** each independently represent a hydrogen atom, a (C₂-C₆) alkanoyl group, a (C₁-C₄) alkyl group, a (C₂-C₄) alkenyl group or a phenyl (C₁-C₄) alkyl group in which the phenyl is unsubstituted or substituted by halogen, (C₁-C₄) alkyl or (C₁-C₄) alkoxy, or a salt thereof; or
(c) deprotecting a compound of formula IV 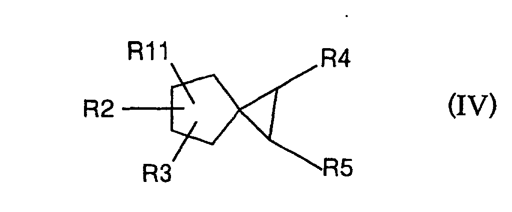 wherein: **R2, R3, R4, R5** are as defined above, and **R11** is: 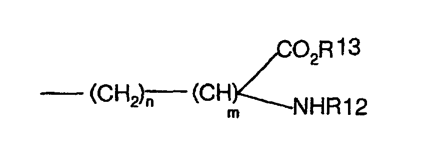 wherein:
   n is 0-3, m is 0 or 1, with the proviso that, when m = 0, then n = 0 and the groups - CO₂R13 and NHR12 are directly attached to the ring, **R13** represents a hydrogen atom or a carboxyl protecting group, or a salt thereof, and **R12** represents a hydrogen atom or a nitrogen protecting group;
   whereafter, if necessary and/or desired:
   (i) resolving the compound of Formula I;
   (ii) converting the compound of Formula I into a non-toxic metabolically-labile ester or amide thereof; and/or;
   (iii) converting the compound of Formula I or a non-toxic metabolically-labile ester or amide thereof into a pharmaceutically acceptable salt thereof.

The protection of carboxylic acid and amine groups is generally described in McOmie, Protecting Groups in Organic Chemistry, Plenum Press, NY, 1973, and Greene and Wuts, Protecting Groups in Organic Synthesis, 2nd. Ed., John Wiley & Sons, NY, 1991. Examples of carboxyl protecting groups include alkyl groups such as methyl, ethyl, *t*-butyl and *t*-amyl; aralkyl groups such as benzyl, 4-nitrobenzyl, 4-methoxybenzyl, 3,4-dimethoxybenzyl, 2,4-dimethoxybenzyl, 2,4,6-trimethoxybenzyl, 2,4,6-trimethylbenzyl, benzhydryl and trityl; silyl groups such as trimethylsilyl and *t*-butyldimethylsilyl; and allyl groups such as allyl and 1-(trimethylsilylmethyl)prop-1-en-3-yl. Examples of amine protecting groups include acyl groups, such as groups of formula R^{11a} CO in which R^{11a} represents (C₁-C₆) alkyl, (C₃-C₁₀) cycloalkyl, phenyl (C₁-C₆) alkyl, phenyl, (C₁-C₆) alkoxy, phenyl (C₁-C₆) alkoxy, or a (C₃-C₁₀) cycloalkoxy, wherein a phenyl group may optionally be substituted by one or two substituents independently selected from amino, hydroxy, nitro, halogeno, (C₁-C₆) alkyl, (C₁-C₆) alkoxy, carboxyl, (C₁-C₆) alkoxycarbonyl, carbamoyl, (C₁-C₆) alkanoylamino, (C₁-C₆) alkylsulphonylamino, phenylsulphonylamino, toluenesulphonylamino, and (C₁-C₆) fluoroalkyl.

The compounds of Formula II are conveniently hydrolyzed in the presence of an acid, such as hydrochloric acid or sulfuric acid, or a base, such as an alkali metal hydroxide, for example sodium hydroxide. The hydrolysis is conveniently performed in an aqueous solvent such as water and at a temperature in the range of 50 to 200 °C.

he compounds of Formula III are conveniently hydrolyzed in the presence of a base, for example an alkali metal hydroxide such as lithium, sodium or potassium hydroxide, or an alkaline earth metal hydroxide such as barium hydroxide. Suitable reaction media include water. The temperature is conveniently in the range of from 50 to 150 °C.

The compounds of Formula IV may be deprotected by a conventional method. Thus, an alkyl carboxyl protecting group may be removed by hydrolysis. The hydrolysis may conveniently be performed by heating the compound of Formula V in the presence of either a base, for example an alkali metal hydroxide such as lithium, sodium or potassium hydroxide, or an alkaline metal hydroxide, such as barium hydroxide, or an acid such as hydrochloric acid. The hydrolysis is conveniently performed at a temperature in the range from 10 to 300 °C. An arylalkyl carboxyl protecting group may conveniently be removed by hydrogenolysis. The hydrogenolysis may conveniently be effected by reacting the compound of Formula V with hydrogen in the presence of a Group VIII metal catalyst, for example a palladium catalyst such as palladium on charcoal. Suitable solvents for the reaction include alcohols such as ethanol. The reaction is conveniently performed at a temperature in the range from 0 to 100 °C. An acyl, amine protecting group is also conveniently removed by hydrolysis, for example as described for the removal of an alkyl carboxyl protecting group.

The compounds of Formula II may be prepared by reacting a compound of formula V with an alkali metal cyanide, such as lithium, sodium or potassium cyanide, and either ammonium carbonate in an aqueous alcohol, such as aqueous ethanol, or with an ammonium halide, such as ammonium chloride, conveniently in the presence of ultrasound. If the reaction is conducted with ammonium carbonate, the reaction is conveniently performed at a temperature in the range from 35 °C to 150 °C. If desired, the compounds of Formula II may then be alkylated, for example using a compound of formula **R**C1, wherein: **R** is (C₁-C₆) straight or branched chain alkyl, or (C₁-C₆) alkanoyl group. The alkylated compounds may be separated into their diastereomers. If the reaction is conducted with an ammonium halide in the presence of ultrasound, the ammonium halide is mixed with chromatography grade alumina in the presence of a suitable diluent such as acetonitrile. The mixture is then irradiated with ultrasound, whereafter the compound of Formula V is added, and the mixture is again irradiated. The alkali metal cyanide is then added, followed by further irradiation with ultrasound. 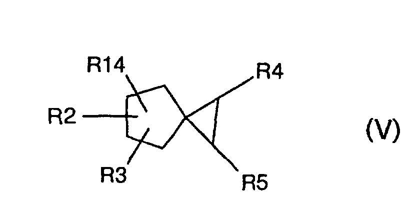 wherein:
**R2, R3, R4, R5** are as defined above, and **R14** is =O, or -(CH₂)ₙ-CHO, wherein: n is 0-3,

Individual isomers of compounds of Formula II may be made by reacting a compound of the Formula V with the stereoisomers of the chiral agent (S) and (R)-phenylglycinol and a reactive cyanide such as trimethylsilyl cyanide.

The compounds of Formula III may be prepared by reacting a compound of Formula V with an alkali metal cyanide, such as lithium, sodium or potassium cyanide, and ammonium carbonate or ammonium carbamate. Convenient solvents include water, dilute ammonium hydroxide, alcohols such as methanol, aqueous methanol and aqueous ethanol. Conveniently the reaction is performed at a temperature in the range of from 10 to 150 °C. If desired, the compounds of Formula III may then be N-alkylated, for example using an appropriate compound of formula **R9** Cl and/or **R10** Cl.

Compounds of the formula V, wherein **R14** is =O or -(CH₂)ₙ-CHO, can be prepared:
(i) by cycloaddition of compound of the formula VI with ethyl acrylate, followed by hydrolysis of the ester group; 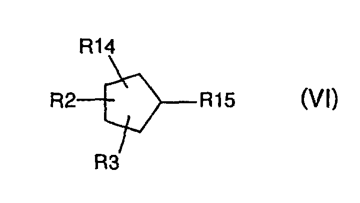 wherein: **R2, R3,** and **R14** are as defined above, and **R15** = Cl, Br, or I, or
(ii) by cyclopropanation or 2 + 2 cycloaddition of the compound of formula (VII) 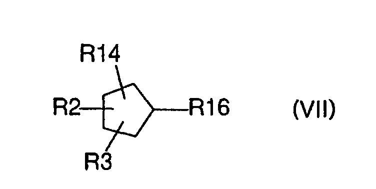 wherein: **R2, R3,** and **R14** are as defined above, and **R16** is =**CHR17**, wherein: R17 is H, alkyl, aryl, halo, heterocyclic, alkyl ester (C₁-C₃) or aryl ester or alkyl aryl ester, or
(iii) by ring closure of the diester VIII or IX under Dieckmann condensation conditions followed by further manipulations of the resulting compounds within the knowledge of a worker skilled in the art; 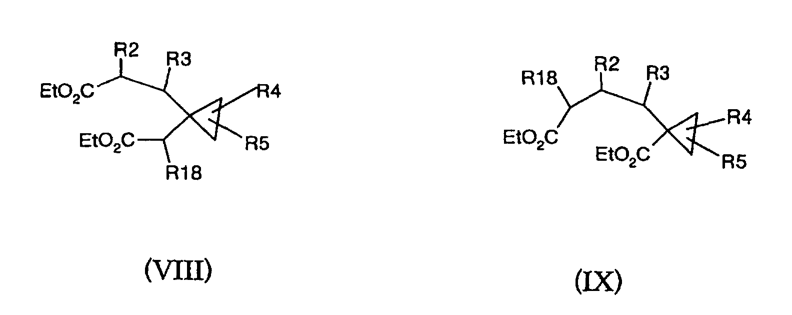 wherein:
   R4, and R5 are as defined above, R2, R3 and R18 is H, halo, alkyl, cycloalkyl, aryl or heterocycle, or when taken together, **R2** and **R3** can form a cycloalkyl (3-6 carbon atoms), heterocycle, an aromatic ring or heteroaromatic ring, or when taken together, R18 and R2 can form a cycloalkyl (3-6 carbon atoms), heterocycle. With the proviso, that (i) at least one of R2, R3 or R18 must be H or (ii) when, R2 and
   **R3,** taken together to form a cycloalkyl (3-6 carbon atoms), heterocycle, an aromatic ring or heteroaromatic ring, then **R18** is H or (iii) when, **R18** and **R2** are taken together to form a cycloalkyl (3-6 carbon atoms), heterocycle, an aromatic ring or heteroaromatic then **R3** is H.

Compounds of the formula V, when **R14** is -(CH₂)ₙ-CHO, may also be prepared via Wittig reaction of compound V where **R14** is =O, with appropriate Wittig salt, followed by further manipulations of the resulting compounds within the knowledge of a worker skilled in the art.

Compounds of the formula VII, wherein: **R14** is =O can be prepared:
(a) by condensation of the compound of formula X with aldehyde of the t ypeHCOR17, 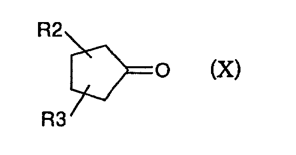 wherein: **R2, R3** and **R17** are as defined above, or
(b) by Wittig reaction of the compound of formula XI with appropriate Wittig reagent, 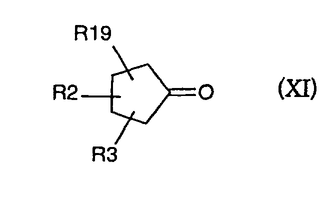 wherein: **R19** is =O, **R2** and **R3** are as defined above, or
   Compounds of the formula VII, wherein: **R14** is -(CH₂)ₙ-CHO, wherein: n is 0-3 may be prepared by Wittig reaction of compound of formula XII, 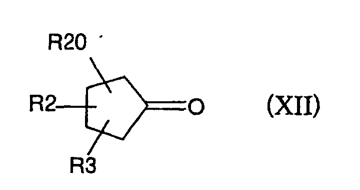 wherein:
   **R20** is -(CH₂)ₙ-CHO, **R2** and **R3** are as defined above, in this case the aldehyde group is protected with an appropriate protecting group before treating the compound XII with appropriate Wittig reagent. The protecting group can be removed after the Wittig reaction to obtain free aldehyde. The protecting groups for carbonyl group are well known to the worker skilled in the art, and examples of these groups can be found in Greene, T.W. and Wuts, P. G. M., *Protective Groups in Organic Synthesis,* Second edition; Wiley-Interscience: 1991; Chapter 4.

Compounds of formulae XI and XII are commercially available or may be synthesized by standard reactions by a person expert in the art.

Many of the intermediates described herein, for example the compounds of Formula II, III and IV are believed to be novel, and are provided as further aspects of the invention.

### Biological and Therapeutic Activity Of Compounds Of Formula (I)

The compounds of formula I of the present invention are agonists or antagonists at certain metabotropic excitatory amino acid receptors (mGluRs). Therefore, another aspect of the present invention is a method of affecting mGluRs in mammals, which comprises administering to a mammal requiring modulated excitatory amino acid neurotransmission a pharmacologically-effective amount of a compound of Formula I. The term "pharmacologically-effective amount" is used to represent an amount of the compound of the invention that is capable of affecting the mGluRs. By affecting, a compound of the invention is acting as an agonist or antagonist. When a compound of the invention acts as an agonist, the interaction of the compound with the excitatory amino acid receptor mimics the response of the interaction of this receptor with its natural ligand (i.e. L-glutamate). When a compound of the invention acts as an antagonist, the interaction of the compound with the excitatory amino acid receptor blocks the response of the interaction of this receptor with its natural ligand (i.e. L-glutamate).

The particular dose of compound administered according to this invention will, of course, be determined by the particular circumstances surrounding the case, including the compound administered, the route of administration, the particular condition being treated, and similar considerations. The compounds can be administered by a variety of routes including oral, rectal, transdermal, subcutaneous, intravenous, intramuscular, or intranasal routes. Alternatively, the compound may be administered by continuous infusion. A typical daily dose will contain from about 0.001 mg/kg to 100 mg/kg of the active compound of this invention. Preferably, daily doses will be 0.05 mg/kg to 50 mg/kg, more preferably from 0.1 mg/kg to 20 mg/kg.

A variety of physiological functions have been shown to be subject to influence by excessive or inappropriate stimulation of excitatory amino acid transmission. The Formula I compounds of the present invention are believed (through their interactions at the mgluRs) to have the ability to treat a variety of neurological disorders in mammals associated with this condition, including acute neurological disorders such as cerebral deficits subsequent to cardiac bypass surgery and grafting, cerebral ischemia (e.g. stroke and cardiac arrest), spinal cord trauma, head trauma, perinatal hypoxia, and hypoglycemic neuronal damage. The Formula I compounds are believed to have the ability to treat a variety of chronic neurological disorders, such as Alzheimer's disease, Huntington's Chorea, amyotrophic lateral sclerosis, AIDS-induced dementia, ocular damage and retinopathy, cognitive disorders, and idiopathic and drug-induced Parkinson's. The present invention also provides methods for treating these disorders which comprises administering to a patient in need thereof an effective amount of a compound of Formula I.

The Formula I compounds of the present invention (through their interactions at the mgluRs) are also believed to have the ability to treat a variety of other neurological disorders in mammals that are associated with glutamate dysfunction, including muscular spasms, convulsions, migraine headaches, urinary incontinence, psychosis, drug tolerance, withdrawal, and cessation (i.e. opiates, benzodiazepines, nicotine, cocaine, or ethanol), smoking cessation, anxiety and related disorders (e.g. panic attack), emesis, brain edema, chronic pain, sleep disorders, Tourette's syndrome, attention deficit disorder, and tardive dyskinesia. Therefore, the present invention also provides methods for treating these disorders which comprise administering to a patient in need thereof an effective amount of the compound of Formula I.

The Formula I compounds of the present invention (through their interactions at the mgluRs) are also believed to have the ability to treat a variety of psychiatric disorders, such as schizophrenia, anxiety and related disorders (e.g. panic attack), depression, bipolar disorders, psychosis, and obsessive compulsive disorders. The present invention also provides methods for treating these disorders which comprises administering to a patient in need thereof an effective amount of a compound of Formula I.

### Functional Assays Employing Cloned Subtypes of Metabotropic Receptors

The pharmacological properties of the compounds of the present invention can be determined *via* appropriate functional assays using recombinant metabotropic glutamate receptors. For example adenylate cyclase assays or phosphatidylinositol hydrolysis assays, performed using standard procedures, can be used to determine agonist or antagonist activity towards mGluRs.

In general assay methods include monitoring of adenylate cyclase activity and phosphatidyl inositol hydrolysis in a cell line that expresses the appropriate mGluR, including but not limited to CHO cell lines.

### (a) Adenylate Cyclase Activity

Adenylate cyclase activity is determined in initial experiments in transfected mammalian cells, using standard techniques. See, e.g., N. Adham, *et al*., Supra; R. L. Weinshank, *et al.* Proc. Natl. Acad. Sci. (USA), 89:3630-3634 (1992), and the references cited therein.

Mammalian cells (the cell line AV12-664 is especially preferred) are stably transfected with a plasmid comprising the cloned metabotropic glutmate receptor. The cells are maintained in aa appropriate medium, for example one consisting of Dulbecco's Modified Eagle's Medium (DMEM) containing 5% dialyzed fetal calf serum, 10 mM HEPES buffer (pH 7.3), 1 mM sodium pyruvate, 1 mM glutamine, and 200 µ.g/mL hygromycin.

For the assay the cells are disassociated from stock culture flasks with trypsin, and plated in 24-well plastic tissue culture dishes (15 mm wells) at a density of 500,000-700,000 cells per well using the same culture medium. After a twenty four hour incubation in a humidified CO₂ incubator, the cell monolayers are washed with buffer (for example Dulbecco's phosphate-buffered saline containing 0.5 mM IBMX and 3 mM glucose) and then incubated in the same buffer at 37 °C for 30 minutes. The monolayers are then washed with six exchanges of buffer.

Test compound(s) and forskolin, or forskolin alone, dissolved in buffer, are added after the final wash. After incubating for 20 minutes at 37 °C, 0.5 mL of 8 mM EDTA is added to each well. The plates are then placed in a boiling water bath for about four minutes. The supernatant fluids are recovered from the wells and lyophilized. Cyclic AMP (cAMP) determinations are carried out on the lyophilized samples using commercially available radioimmunoassay kits, following the manufacturer's instructions. The cAMP levels in wells containing test compound(s) are then compared to the forskolin controls.

### (b) Phosphatidylinositol Assay

Phosphatidylinositol hydrolysis is measured in clonal cell lines (for example AV12) harbouring a plasmid expressing the cloned metabotropic glutamate receptor in response to addition of glutamate agonists, as a functional assay for metabotropic glutamate receptor activity according to D. Schoepp, Trends in Pharmaceutical Sciences, 11:508, 1990.

Twenty four well tissue culture vessels are seeded with approximately 250,000 cells per well in an appropriate medium for example Dulbecco's Minimal Essential Media (D-MEM) (in the absence of glutamic acid) containing 2 mM glutamine and 10% dialyzed fetal calf serum. After 24 hours growth at 37 °C, the media is removed and replaced with fresh media containing four microcuries of [³ H]myoinositol per well and the cultures are incubated a further 16 to 20 hours. The media is then removed and the cells in each well are washed with serum free medium containing 10 mM lithium chloride, 10 mM myoinositol, and 10 mM HEPES (2 x 1 mL washes). After the final wash, 0.5 mL of washing solution is added containing the appropriate concentration(s) of test compound(s).

If the particular assay is also testing antagonists, a ten minutes incubation is performed prior to antagonist induction. Cells are incubated for about one hour at 37 °C. in 95%:5% O₂ :CO₂ or as appropriate for time course. The reactions are terminated by removing media and adding 1 mL of cooled 1:1 acetone:methanol followed by incubation on ice for a minimum of twenty minutes.

These extracts are then collected and placed in 1.5 mL centrifuge tubes. Each well is washed with 0.5 mL water and this wash is added to the appropriate extract. After mixing and centrifugation, each aqueous supernatant is processed by chromatography on a QMA SEP-PAK® column, which is prewetted and equilibrated by passing 10 mL of water, followed by 8 mL of 1M triethylammonium hydrogen carbonate (TEAB), followed by 10 mL of water through the column.

The assay supernatants containing the water soluble [³H]inositol phosphate are passed over the columns. This is followed by a 10 mL water wash and a 4 mL wash with 0.02 M TEAB to remove [³H]inositol precursors. [³H]inositol phosphate is eluted with 4 mL of 0.1 M TEAB into scintillation vials and counted in the presence of scintillation cocktail. Total protein in each sample is measured using standard techniques. Measurements are taken as the amount of [³H]inositol phosphate released per milligram of protein.

The assays are carried out in the absence and in the presence of the compound being tested. The measurements of [³H]inositol phosphate per milligram of protein are compared in order to confirm agonist and antagonist activity of the compound being tested.

These types of assays, employing cell lines expressing different subtype of cloned metabotropic receptors, may be used to determine which compounds have selective affinity in that they modulate one subtype of receptor with a greater activity than another subtype.

### (c) Testing in Chinese hamster cell lines

The Chinese hamster ovary cell lines expressing mGluR_{1α}, mGlu R₂ and mGluR_{4α} receptors have been described previously (Amarori and Nakanishi, Neuron 8, 757-765, 1992; Tanabe *et al*., Neuron 8, 169-179, 1992, and J. Neurochem. 63, 2038-2047, 1993). They are maintained at 37 °C in a humidified 5% CO₂ incubator in Dubecco's Modified Eagle Medium (DMEM) containing a reduced concentration of (S)-glutamine (2mM) and are supplemented with 1% proline, penicillin (100 U/mL), streptomycin (100 mg/mL) and 10% dialyzed fetal calf serum (all GIBCO, Paisley). Two days before assay 1.8 x 10⁶ cells are evenly distributed into the wells of 24 well plates.

Phosphatidylinositol (PI) hydrolysis can be measured as described previously (Hayashi *et al*., Nature 366, 687-690,1992, and J. Neuroscience 14, 3370-3377, 1994). Briefly, the cells are labeled with [³H]inositol (2µ Ci/mL) 24 h prior to the assay. For agonist assays, the cells are incubated with test compound dissolved in phosphate-buffered saline (PBS)-LiCl for 20 min, and agonist activity is determined from the level of ³H-labeled mono-, bis- and tris-inositol phosphates generated, as measured following ion-exchange chromatography, compared with the level generated in the absence of the test compound. For antagonist assays, the cells are preincubated with ligand dissolved in PBS-LiCl for 20 min prior to incubation with test compound and 10 µ M (S)-Glu for 20 min. The antagonist activity is then determined as the inhibitory effect of the (S)-Glu mediated response.

The assay of cyclic AMP formation can be performed as described previously (Hayashi *et al*., 1992, 1994). Briefly, the cells are incubated for 10 min in PBS containing test coumpound and 10 µ M forskolin and 1 mM 3-isobutyl-1-methylxanthine (IBMX) (both Sigma, St. Louis, MO, USA). The agonist activity is then determined as the inhibitory effect on the forskolin-induced cyclic AMP formation. For antagonist assay, the cells are preincubated with ligand dissolved in PBS containing 1 mM IBMX for 20 min prior to a 10 min incubation in PBS containing test compound, 20 µ M(mGlu2) or 50 µ M (mGlu4a) (S)-Glu, 10 µ M forskolin and 1 mM IBMX. The antagonist activity is then determined as the potentiating effect on the forskolin-induced cyclic AMP formation.

Testing for demonstration of the pharmacological activity of certain compounds on representative mGlu receptor subtypes can be performed using Sprague Dawley rat tissues.

Phosphatidylinositol (PI) hydrolysis can be measured as described below: Briefly, cross-chopped slices are prepared from neonatal Sprague Dawley rat tissue (age: p7-p14). The slices are pre-labelled with [³H] myo-inositol. Following pre-labelling, the slices are incubated with the test drugs and standard (known Group I agonists *i.e*. ACPD) for a period of 45 minutes. The incubation is terminated by the addition of chloroform/methanol/HCl (100:200:2). The resulting mixture is separated into two phases by the addition of chloroform and distilled water. The aqueous fraction is applied to ion exchange columns, and inositol phosphates are eluted using 800 mM Ammonium Formate/100 mM Formic Acid. The eluent is then analyzed using liquid scintillation counting. The amount of inositol phosphate accumulation is expressed as a percentage of that induced by ACPD.

The assay of cyclic AMP formation can be performed as described previously (Tovey *et al*., *Clinica Chimica Acta,* 56, 221-234, 1974). The assay can be modelled on the cyclic AMP assay kit available from Amersham, which in turn, is based on the assay created by Tovey *et al*. Briefly, samples are prepared from Sprague Dawley rat (225-250g) cortical slices. Slices are incubated with the drug, and then challenged with forskolin to induce cyclic AMP release. Following termination of the reaction by boiling, the slices are homogenized and centrifuged. Samples of supernatant are then incubated for 2-3 hours with a known quantity of [³H]cAMP and a binding protein. When the incubation is complete, the bound cyclic AMP is separated from the free cyclic AMP by centrifugation with charcoal. The resulting supernatant (containing free cyclic AMP) is then analyzed by liquid scintillation counting. The amount of unbound cyclic AMP can be calculated from a standard curve previously determined with various samples of free cyclic AMP.

In performing such experiments with some of the compounds of the present invention, it has been demonstrated that some compounds of the present invention act as modulators of the cAMP-linked metabotropic glutamate receptors, while showing less activity with phosphatidylinositol-linked metabotropic glutamate receptors and *vice versa*.

### Administration of Compounds of Formula (I)

According to another aspect, the present invention provides a method of modulating one or more metabotropic glutamate receptor functions in a warm-blooded mammal which comprises administering an effective amount of a compound of Formula I, or a non-toxic metabolically-labile ester or amide thereof, or a pharmaceutically acceptable salt thereof.

The compounds of the present invention are preferably formulated prior to administration. Therefore, another aspect of the present invention is a pharmaceutical formulation comprising a compound of Formula I and a pharmaceutically-acceptable carrier, diluent, or excipient. The present pharmaceutical formulations are prepared by known procedures using well-known and readily available ingredients. In making the compositions of the present invention, the active ingredient will usually be mixed with a carrier, or diluted by a carrier, or enclosed within a carrier, and may be in the form of a capsule, sachet, paper, or other container. When the carrier serves as a diluent, it may be a solid, semi-solid, or liquid material that acts as a vehicle, excipient, or medium for the active ingredient.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also provides pharmaceutical compositions containing compounds as disclosed in the claims in combination with one or more pharmaceutically acceptable, inert or physiologically active, diluent or adjuvant. The compounds of the invention can be freeze-dried and, if desired, combined with other pharmaceutically acceptable excipients to prepare formulations for administration. These compositions may be presented in any form appropriate for the administration route envisaged. The parenteral and the intravenous route are the preferential routes for administration.

Compounds of the general Formula I may be administered orally, topically, parenterally, by inhalation or spray or rectally in dosage unit formulations containing conventional non-toxic pharmaceutically acceptable carriers, adjuvants and vehicles. The term parenteral as used herein includes subcutaneous injections, intravenous, intramuscular, intrastemal injection or infusion techniques. In addition, there is provided a pharmaceutical formulation comprising a compound of general Formula I and a pharmaceutically acceptable carrier. One or more compounds of general Formula I may be present in association with one or more non-toxic pharmaceutically acceptable carriers and/or diluents and/or adjuvants and if desired other active ingredients. The pharmaceutical compositions containing compounds of general Formula I may be in a form suitable for oral use, for example, as tablets, troches, lozenges, aqueous or oily suspensions, dispersible powders or granules, emulsions, hard or soft capsules, or syrups or elixirs.

Compositions intended for oral use may be prepared according to any known to the art for the manufacture of pharmaceutical compositions and such compositions may contain one or more agents selected from the group consisting of sweetening agents, flavouring agents, colouring agents and preserving agents in order to provide pharmaceutically elegant and palatable preparations. Tablets contain the active ingredient in admixture with non-toxic pharmaceutically acceptable excipients that are suitable for the manufacture of tablets. These excipients may be for example, inert diluents, such as calcium carbonate, sodium carbonate, lactose, calcium phosphate or sodium phosphate: granulating and disintegrating agents for example, corn starch, or alginic acid: binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain active materials in admixture with excipients suitable for the manufacture of aqueous suspensions. Such excipients are suspending agents, for example sodium carboxylmethylcellulose, methyl cellulose, hydropropylmethylcellulose, sodium alginate, polyvinylpyrrolidone, gum tragacanth and gum acacia: dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example hepta-decaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl- p-hydroxy benzoate, one or more coloring agents, one or more flavoring agents or one or more sweetening agents, such as sucrose or saccharin.

Oily suspensions may be formulated by suspending the active ingredients in a vegetable oil, for example peanut oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavouring agents may be added to provide palatable oral preparations. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavouring and colouring agents, may also be present.

Pharmaceutical compositions of the invention may also be in the form of oil-in-water emulsions. The oil phase may be a vegetable oil, for example olive oil or peanut oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol, anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleaginous suspension. This suspension may be formulated according to known art using those suitable dispersing or wetting agents and suspending agents that have been mentioned above. The sterile injectable preparation may also be a sterile injectable solution or a suspension in a non-toxic parentally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

The compound(s) of the general Formula I may be administered, together or separately, in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Compound(s) of general Formula I may be administered, together or separately, parenterally in sterile medium. The drug, depending on the vehicle and concentration used, can either be suspended or dissolved in the vehicle. Advantageously, adjuvants such as local anaesthetics, preservatives and buffering agents can be dissolved in the vehicle.

The dosage to be administered is not subject to defined limits, but it will usually be an effective amount. It will usually be the equivalent, on a molar basis of the pharmacologically active free form produced from a dosage formulation upon the metabolic release of the active free drug to achieve its desired pharmacological and physiological effects. The compositions are preferably formulated in a unit dosage form, each dosage containing from 0.05 to 100 mg, more usually about 1.0 to 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range. For examples, dosages per day normally fall within the range of 0.01 to 30 mg/kg of body weight. A typical daily dose will contain from 0.01 mg/kg to 100 mg/kg of the active compound of this invention. Preferably, daily doses will be 0.05 mg/kg to 50 mg/kg, more preferably from 0.1 mg/kg to 25 mg/kg. In the treatment of adult humans, the range of 0.1 to 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

The compositions are preferably formulated in a unit dosage form, each dosage containing from 5 mg to 500 mg, more preferably about 25 mg to about 300 mg of the active ingredient. The term "unit dosage form" refers to a physically discrete unit suitable as unitary dosages for human subjects and other mammals, each unit containing a predetermined quantity of active material calculated to produce the desired therapeutic effect, in association with a suitable pharmaceutical carrier, diluent, or excipient. The following formulation examples are illustrative only and are not intended to limit the scope of the invention in any way.

### Formulation 1

Hard gelatin capsules are prepared using the following ingredients:

| | **Quantity (mg/capsule)** |
|---|---|
| Active Ingredient | 250 |
| Starch, dried | 200 |
| Magnesium stearate | 10 |
| Total | 460 |

The above ingredients are mixed and filled into hard gelatin capsules in 460 mg quantities.

### Formulation 2

A tablet is prepared using the ingredients below:

| | **Quantity (mg/tablet)** |
|---|---|
| Active Ingredient | 250 |
| Cellulose, microcrystalline | 400 |
| Silicon dioxide, fumed | 10 |
| Stearic acid | 5 |
| Total | 665 |

The components are blended and compressed to form tablets each weighing 665 mg.

### Formulation 3

An aerosol solution is prepared containing the following components:

| | Weight % |
|---|---|
| Active Ingredient | 0.25 |
| Ethanol | 29.75 |
| Propellant 22 (Chlorodifluoromethane) | 70.00 |
| | |
| Total | 100 |

The active compound is mixed with ethanol and the mixture added to a portion of the Propellant 22, cooled to -30 °C and transferred to a filling device. The required amount is then fed to a stainless steel container and diluted with the remainder of the propellant. The valve units are then fitted to the container.

### Formulation 4

Tablets each containing 60 mg of active ingredient are made as follows:

| | **Quantity (mg/tablet)** |
|---|---|
| Active Ingredient | 60 |
| Starch | 45 |
| Microcrystalline cellulose | 35 |
| Polyvinylpyrrolidone | 4 |
| Sodium carboxymethyl starch | 4.5 |
| Magnesium stearate | 0.5 |
| Talc | 1.0 |
| Total | 150 |

The active ingredient, starch, and cellulose are passed through a No. 45 mesh U.S. sieve and mixed thoroughly. The solution of polyvinylpyrrolidone is mixed with the resultant powders that are then passed through a No. 14 mesh U.S. sieve. The granules so produced are dried at 50□C and passed through a No. 18 mesh U.S. sieve. The sodium carboxymethyl starch, magnesium stearate, and talc, previously passed through a No. 60 mesh U.S. sieve, are then added to the granules which, after mixing, are compressed on a tablet machine to yield tablets each weighing 150 mg.

### Formulation 5

Capsules each containing 80 mg medicament are made as follows:

| | **Quantity (mg/capsule)** |
|---|---|
| Active Ingredient | 80 |
| Starch | 59 |
| Microcrystalline cellulose | 59 |
| Magnesium stearate | 2 |
| Total | 200 |

The active ingredient, cellulose, starch, and magnesium stearate are blended, passed through a No. 45 sieve, and filled into hard gelatin capsules in 200 mg quantities.

### Formulation 6

Suppositories each containing 225 mg of active ingredient may be made as follows:

| | **Quantity (mg/suppository)** |
|---|---|
| Active Ingredient | 225 |
| Saturated fatty acid glycerides | 2000 |
| Total | 2225 |

The active ingredient is passed through a No. 60 mesh U.S. sieve and suspended in the saturated fatty acid glycerides previously melted using the minimum heat necessary. The mixture is then poured into a suppository mold of nominal 2 g capacity and allowed to cool.

### Formulation 7

Suspensions each containing 50 mg of medicament per 5 mL dose are made as follows:

| | |
|---|---|
| Active Ingredient | 50 mg |
| Sodium carboxylmethyl cellulose | 50 mg |
| Syrup | 1.25 mL |
| Benzoic acid solution | 0.10 mL |
| Flavour | q.v. |
| Color | q.v. |
| Purified water to total | 5 mL |

The medicament is passed through a No. 45 mesh U.S. sieve and mixed with the sodium carboxymethyl cellulose and syrup to form a smooth paste. The benzoic acid solution, flavor and color are diluted with some of the water and added, with stirring. Sufficient water is then added to produce the required volume.

### Formulation 8

An intravenous formulation may be prepared as follows:

| | Quantity |
|---|---|
| Active Ingredient | 100 mg |
| Mannitol | 100 mg |
| 5 N Sodium hydroxide | 200 mL |
| Purified water to total | 5 mL |

### Formulation 9

A topical formulation may be prepared as follows:

| | Quantity |
|---|---|
| Active Ingredient | 1-10 g |
| Emulsifying Wax | 30 g |
| Liquid Paraffin | 20 g |
| White soft paraffin | 100 g |

The white soft paraffin is heated until molten. The liquid paraffin and emulsifying wax are incorporated and stirred until dissolved. The active ingredient is added and stirring is continued until dispersed. The mixture is then cooled until solid.

### Formulation 10

Sublingual or buccal tablets, each containing 10 mg of active ingredient, may be prepared as follows:

| | Quantity (mg/tablet) |
|---|---|
| Active Ingredient | 10.0 |
| Glycerol | 210.5 |
| Water | 143.0 |
| Sodium Citrate | 4.5 |
| Polyvinyl Alcohol | 26.5 |
| Polyvinylpyrrolidone | 15.5 |
| Total | 410.0 |

The glycerol, water, sodium citrate, polyvinyl alcohol, and polyvinylpyrrolidone are admixed together by continuous stirring and maintaining the temperature at about 90 °C. When the polymers have gone into solution, the solution is cooled to about 50°-55 °C. and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts.

The construction and use of transdermal patches for the delivery of pharmaceutical agents is well known in the art (see, for example, U.S. Pat. No. 5,023,252 issued Jun. 11, 1991). Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Pat. No. 5,011,472, issued Apr. 30, 1991.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions that can transiently open the blood-brain barrier.

### EXAMPLES

The following Examples illustrate the invention. The following abbreviations are used in the Examples: EtOAc, ethyl acetate; THF, tetrahydrofuran; EtOH, ethanol; TLC, thin layer chromatography; GC, gas chromatography; HPLC, high pressure liquid chromatography; m-CPBA, m-chloroperbenzoic acid; Et₂O, diethyl ether; DMSO, dimethyl sulfoxide; DBU, 1,8-diazabicyclo-[5.4.0]undec-7-ene, MTBE, methyl t-butyl ether; and FDMS, field desorption mass spectrometry.

### Example 1: Spiro[2.4] heptane-4-amino-1,4-dicarboxylic acid Isomers (7) and (8)

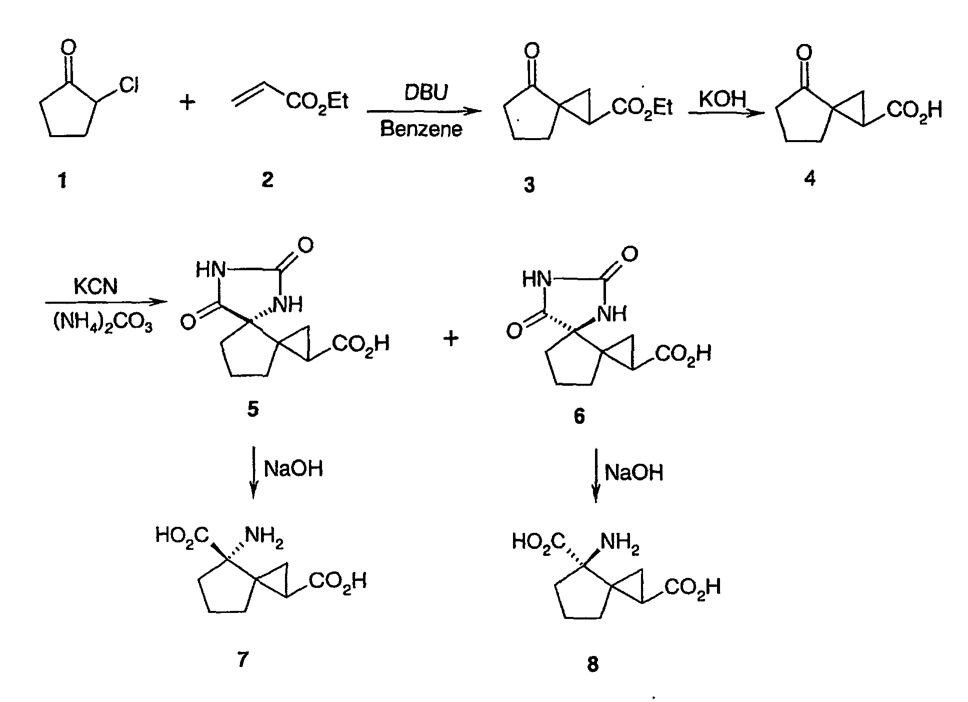

### Preparation of Ethyl spiro[2.4]heptane-4-oxo-1-carboxylate (3)

To a solution of 2-chlorocyclopentanone (1) (10 g, 0.084 mol) and ethyl acrylate (2) (16.8 g, 0.168 mol) in benzene (50 mL), DBU (15 mL, 0.10 mol) was added at room temperature (RT). After stirring for 2 h and 30 minutes, the mixture was poured into water. The mixture was extracted with EtOAc, washed with H₂O, dried with MgSO₄, and evaporated to dryness. The residue was purified by chromatography on a silica gel column with 5-75% EtOAc/hexanes as the eluent. Product (3) was obtained as a colourless oil (5.99 g, 40%).

### Preparation of [2.4] heptane-4-oxo-1-carboxylic acid (4)

Compound (3) (5.0 g) was hydrolyzed in KOH (1N, 32.5 mL) and EtOH (200 mL) at RT overnight. The acid was obtained as a solid (100%).

### Preparation of Hydantoins (5) and (6)

Compound (4) 3.92 g was placed in a pressure bottle with KCN, (NH₄)₂CO₃ and 1:1 H₂O:EtOH. The reaction was stirred at 80 °C overnight. The hydantoins were isolated by addition of 6M HCl and the two isomers were separated by chromatography with MeOH/CHCl₃/H₂O/NH₃ (26:70:4:1). Hydantoin 5 comes out of the column first (1.8 g, solid), followed by hydantoin 6 (1.8 g, solid).

### Preparation of Amino Acids (7) and (8)

Hydantoin (5) (800 mg) was dissolved in 2 N NaOH in a sealed pressure tube and is heated to 100 °C for 46 h. The resulting solution was cooled and acidified (6N HCl). The solution was evaporated to dryness and extracted with EtOH. The ethanolic extract was treated with 4 mL of propylene oxide to precipitate the crude amino acid. The amino acids were purified by cation exchange chromatography. 430 mg of compound (7) was derived from hydantoin (5). Following the same procedure as for hydantoin 5, 380 mg of compound 8 was derived from 800 mg of hydantoin (**6**).

Analysis calculated for amino acid (**7**) C₉H₁₃NO₄.H₂O: % C, 49.16; %H, 6.96; %N, 6.45. Found: %C, 49.35; %H, 6.58; %H, 7.19.
Analysis calculated for amino acid (**8**) C₉H₁₃NO₄.H₂O: % C, 53.07; %H, 6.68; %N, 6.88. Found: %C, 53.16; %H, 6.51; %H, 6.95.
¹H NMR (200 MHz, D₂O): δ 5.2-4.9 (m 1H), 4.6-4.2 (m 6H), 3.7, 3.6 (AB system, 2H) ppm.

### Example 2: Spiro [2.4] heptane4-amino-2-phenyl-1,4-dicarboxylic acid Isomers (16 ) and (17)

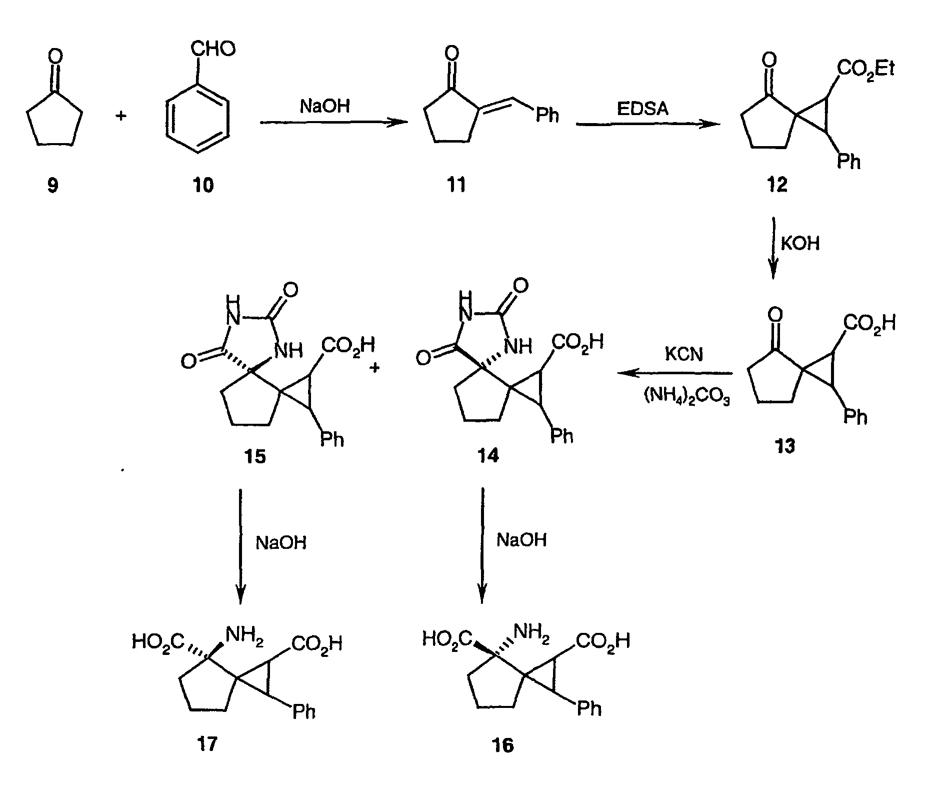

### Preparation of 2-Phenylmethylenecyclopentanone (11)

A mixture of cyclopentanone (9) (6.0g, 0.071 mol), benzaldehyde (10), (7.35 g, 0.069 mol) and NaOH (1N, 66.6 mL) in ether (70 mL) was stirred at room temperature for 64 h. The organic layer was separated from the aqueous layer and washed with water. The residue was purified after evaporation of the solvent by chromatography and the product was obtained in solid form. (7.29 g, 60%).

### Preparation of Ethyl spiro[2.4]heptane-4-oxo-2-phenyl-1-carboxylate (12)

A mixture of (11) (6.82g, 0.04 mol) and EDSA in benzene (50 mL) was heated to reflux overnight. *[EDSA was made as follows: A solution of (Ethoxycarbonylmethyl)dimethyl-sulfonium bromide (13.79g, 0.06 mol) in CHCl*_{*3*} *(50 mL) was treated with K*_{*2*}*CO*_{*3*} *(saturated, 40 mL) for 1 hour at room temperature. The chloroform layer was dried with MgSO4. The solvent was evaporated to give an oil (5.92g, 0.04 mol)]*. After cooling the reaction mixture to room temperature, the organic layer was washed with brine, dried over anhydrous MgSO₄ and concentrated *in vacuo.* Purification by column chromatography (CH₂Cl₂/Hexanes 1:1) afforded 9.96g (95%) of the title compound (**12**) as a solid.

### Preparation of hydantoins (14 and 15) from 12

The ketone ester (**12**) (9.92g) was treated in EtOH (250 mL) with KOH (1N, 45.0 mL) at room temperature overnight. The mixture was neutralized with KHSO₄ and the resulting precipitates were collected by filtration to give a solid (13, 8.95g, 100%). This solid (13, 2.30g, 10.0 mmol) was treated with KCN (1.68g, 25.0 mmol) and (NH₄)₂CO₃ (6.69g, 70 mmol) in H₂O-EtOH (1:1, 20 mL) at 85°C overnight. The resulting mixture was acidified cautiously (6N HCl) and filtered to give a solid containing two isomers. The isomers (**14** and **15**) were separated by chromatography (CHCl₃/MeOH/H₂O/NH₃/H₂O, 70:26:4:1). Hydantoin (**14**) (0.88 g) comes off first and then hydantoin (**15**) (0.63g).

### Preparation of Spiro[2.4]heptan-4-amino-2-phenyl-1,4-dicarboxylic acids (16 and 17)

The hydantoin (**14**) (0.88 g) was hydrolyzed at 136°C in a pressure tube overnight. The resulting solution was cooled and acidified (6N HCl). The solution was evaporated to dryness and extracted with EtOH. The ethanolic extracts were treated with 4 mL of propylene oxide to precipitate the crude amino acid. The crude product was purified by cation ion-exchange chromatography. 210 mg (26%) of (**16**) was obtained. In a similar manner, 50 mg of (**17**) was obtained (9%) from 606 mg of hydantoin (**15**).

Analysis calculated for compound (**17**) (C₁₅H₁₇NO₄.H₂O.0.2NH₄Cl: % C, 63.99; %H, 5.37; %N, 9.33. Found: %C, 58.84; %H, 6.20; %H, 5.21.
¹H NMR (200 MHz, D₂O) δ 7.15 (s 5H), 2.9 (d 1H), 2.65 (d 1H), 2.5-1.6 (m 6H) ppm.

### Example 3: 1-Amino-1-carboxyindan-3-spiro-cyclopropanecarboxylic acid

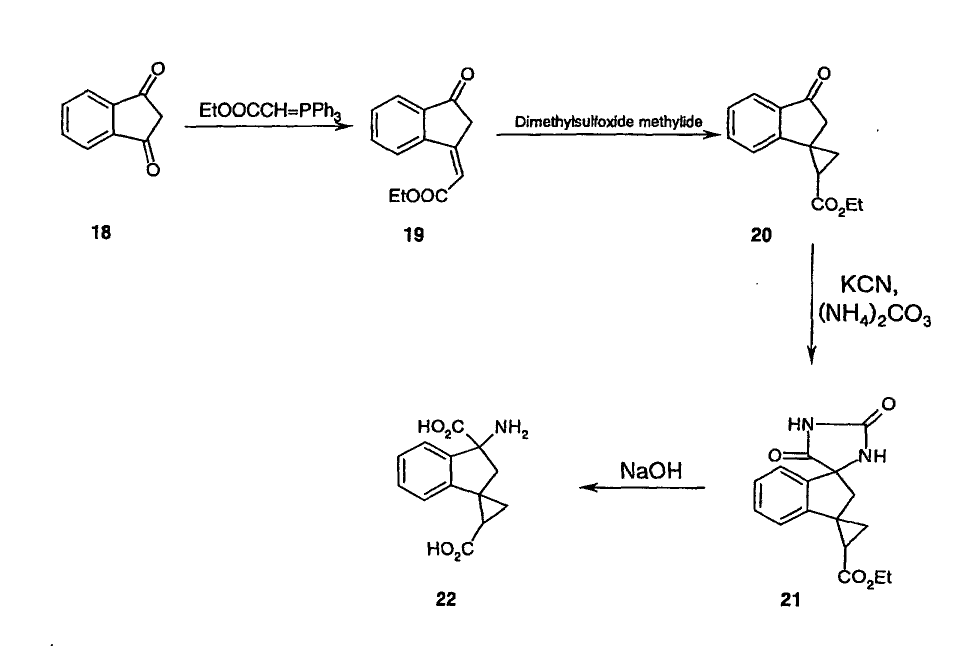

### Preparation of Compound (19)

5g of 1,3 Indandione (**18**) was suspended in toluene (180) mL and (carbethoxymethylene) triphenylphosphorane (13.1 g, 37.63 mmol) was added. The resulting solution was refluxed for 3.5 h. The solution was cooled and the solvent evaporated. The crude mixture was purified by column chromatography (hexane : EtOAc (3:1) yielding 4.38 g of pure compound (19).

### Preparation of Compound (20)

Compound (**19**) (1.12 g, 5.18 mmol) was dissolved in dry THF (20 mL). Dimethylsulfoxide methylide (0.5 M, 12.44 mL, 6.2 mmol) was added dropwise under nitrogen gas. The resulting solution as stirred at room temperature for 2 h. H₂O (10 mL) was added and the mixture extracted with Et₂O (2 x 30 mL). The combined extracts were washed with brine and dried. Column chromatography of the crude mixture using hexanes: EtOAc (3:1) to give compound (20). (0.13 g, 0.55 mmol)

### Preparation of Compound (21)

Compound (**20**) (0.13 g, 0.55 mmol), ethanol (4.5 mL) and 1 N NaOH (0.65 mL) were stirred at 70 °C for 4 hrs. and the solvent was evaporated *in vacuo.* The residue was dissolved in 4 mL (1:1, EtOH: H₂O) and treated with KCN (90 mg) and (NH₄)₂CO₃ (238 mg).The resulting mixture was stirred at 85 °C for 60 h, under pressure. The solution was then cooled to room temperature and acidified with 6N HCl. The ethanol was evaporated and the aqueous solution extracted with EtOAc. The product was used for the next step without any purification.

### Preparation of Compound (22)

Compound (**21**) (crude) was dissolved in 2 M NaOH (5 mL) and the resulting solution was stirred at 150 °C for 24 h under pressure. The solution was then cooled and acidified with 6 N HCl. A precipitate formed which was filtered and washed with water. The filtrate was then concentrated *in vacuo* and put on an anion exchange column using 0.5 M HOAc as an eluent to give 56 mg of compound (**22**) as a white powder. Yield was 31.8% from compound (**20**).

Analysis calculated for compound (**22**): C₁₃H₁₃NO₄: % C, 57.90; %H, 5.69; %N, 5.18. Found: %C, 57.44; %H, 5.39; %H, 4.82.
¹H NMR (200 MHz, D₂O) δ 7.4-7.2 (m, 4H), 2.45 (m, 1H), 1.9-1.7 (m, 2H), 1.05, 0.95 (AB system 2H) ppm.

### Example 4 Spiro[4,2]heptane-1-carboxy-4-glycine Isomers (27) and (028)

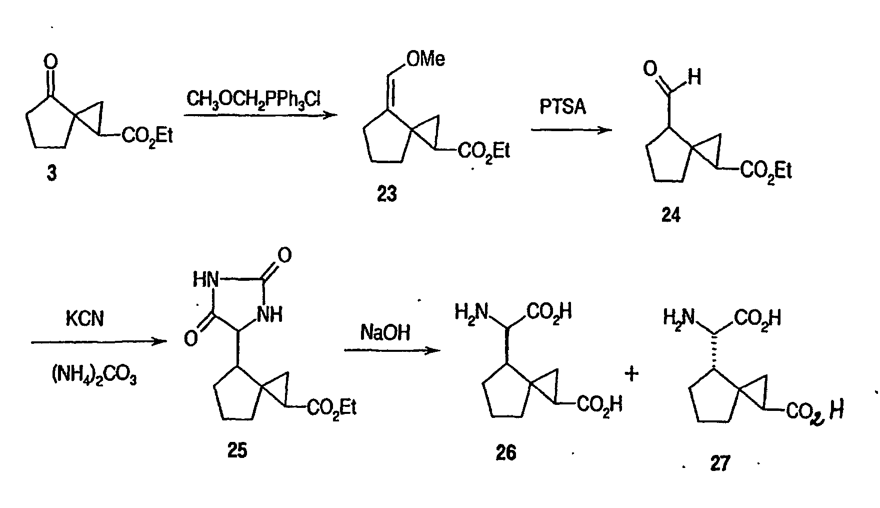

### Preparation of intermediate 23

Sodium bis(trimethylsilyl)amide (26.7 mL) was added to a stirred suspension of (methoxy methyl)triphenylphosphonium chloride (9.5024g) in dry THF (80 mL) at 0 °C under N₂. The resulting red mixture was stirred at 0 °C for 35 minutes, followed by addition of a solution of compound (3) (4.31g) in dry THF (40 mL) over 10 minutes. The reaction mixture was stirred at 0 °C for 2 h and at room temperature for 1 hour. Water (30 mL) was added to the reaction mixture and then the mixture was partitioned between brine (200 mL) and EtOAc (200 mL). The organic phase was washed with brine (2 x 150 mL) and the combined aqueous phase was extracted again with EtOAc (3 x 150 mL). The combined EtOAc extracts were dried and concentrated under *vacuo.* The crude product was purified by column chromatography (hexanes: EtOAc, 9: 1) to obtain 3.11g of compound 23.

### Preparation of Compound 24

Compound 2 (4.4 g) in dioxane-H₂O (90 mL 2:1) was heated to reflux for 2 h, and stirred overnight at room temperature. The reaction mixture was diluted with water and extracted with Et₂O. The organic extracts were washed with NaHCO₃ (50%) followed by water, dried over anhydrous MgSO₄ and concentrated *in vacuo* to give 3.0 g of the compound **24** as an oil.

### Preparation of Compounds 25

A mixture of **24** (3.0 g, 0.015 mol), KCN (2.08 g, 0.032 mol) and (NH₄)₂CO₃ (10.7 g, 0.11 mol) in EtOH-H₂O (30 mL 1:1) was heated at 60 °C overnight. The pH was adjusted to 5 with 6N HCl. The reaction mixture was extracted with EtOAc and the organic solvent was evaporated to give compound 25 as dark oil.

### Preparation of Compound 26 and 27

Compound 25, obtained from the above reaction, was treated with 3N NaOH under reflux conditions overnight. The reaction mixture was acidified with 6 N HCl and then HCl and H₂O were evaporated. The residue was treated with EtOH. Filtration and evaporation of EtOH gave a foam. The foam was submitted to cation-exchange resin chromatography and eluted with 10% pyridine to obtain 250 mg of the amino acid **26** and 14 mg of amino acid **27**.
¹H NMR (200 MHz, D₂O) δ 3.3 (d, 1H), 2.4-2.1 (m, 1H), 1.9-1.3 (m, 7H), 1.05, 0.9 (AB system 2H) ppm.

### Example 5: Testing of Exemplary Compounds:

### Cyclic AMP assay:

### Rationale:

Group II/III metabotropic glutamate receptors (mGluRs) are negatively coupled to adenylate cyclase, and agonists of these receptors lead to a decrease in intracellular cyclic AMP accumulation.

### Method:

The assay has been modeled on the cyclic AMP assay kit available from Amersham. This kit, in turn, is based on the assay created by Tovey *et al*. (1974). Briefly, the samples were prepared from Sprague Dawley rat (225-250g) cortical slices. Slices were incubated with the test compound (drug), and then challenged with forskolin to induce cyclic AMP release. Following termination of the reaction by boiling, the slices were homogenized and centrifuged. Samples of supernatant were then incubated for 2-3 hours with a known quantity of [³H]cAMP and a binding protein. When the incubation was complete, the bound cyclic AMP was separated from the free cyclic AMP by centrifugation with charcoal. The resulting supernatant (containing free cyclic AMP) was then analyzed by liquid scintillation counting. The amount of unbound cyclic AMP was calculated from a standard curve previously determined with various samples of free cyclic AMP.

### Results Interpretation:

If the drugs tested inhibit forskolin-induced cyclic AMP accumulation, they are considered to be Group II/III agonists. Conversely, if they inhibit the decrease in forskolin-induced cyclic AMP accumulation caused by glutamate, they are considered to be Group II/III antagonists.

### Results:

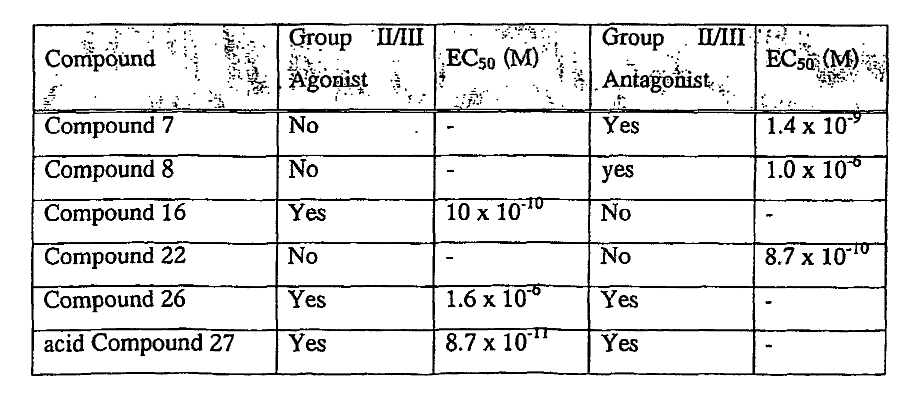

### Phosphatidylinositol Assay

### Rationale:

Group I metabotropic glutamate receptors (mGluRs) are positively coupled on inositol phosphate metabolism. Agonists at these receptors lead to an increase in intracellular free inositol phosphates, while antagonists inhibit the increase in intracellular free inositol phosphate induced by standard agonists (*i.e*. ACPD).

### Method:

Cross-chopped slices were prepared from neonatal Sprague Dawley rat tissue (age: p7-p14). The slices were pre-labelled with [³H] myo-inositol. Following pre-labelling, the slices were incubated with the test compounds and standard (known Group I agonists *i.e*. ACPD) for a period of 45 minutes. The incubation was terminated by the addition of chloroform/methanol/HCl (100:200:2). The resulting mixture was separated into two phases by the addition of chloroform and distilled water. The aqueous fraction was applied to ion exchange columns, and inositol phosphates were eluted using 800 mM Ammonium Formate/100 mM Formic Acid. The eluent was then analyzed using liquid scintillation counting. The amount of inositol phosphate accumulation was expressed as a percentage of that induced by ACPD.

### Results Interpretation:

If the drugs cause an increase in intracellular free inositol phosphate accumulation, they are considered to be Group I agonists. If they inhibit the increase in intracellular free inositol phosphate accumulation induced by ACPD, they are considered to be Group II antagonists.

### Results:

| Compound | Group I Agonist | EC₅₀ (M) | Group I Antagonist | EC₅₀ (M) |
|---|---|---|---|---|
| Compound 7 | No | - | Yes | 2.2 x 10⁻⁷ |
| Compound 8 | No | - | Yes | 9.4 x 10⁻⁸ |
| Compound 16 | No | - | Yes | 1.0 x 10⁻⁷ |
| Compound 22 | No | - | No | - |
| Compound 26 | No | - | Yes | 8.8 x 10⁻⁸ |
| acid Compound 27 | No | - | Yes | 8.4 x 10⁻¹⁰ |

## Claims

1. A compound having the structural formula (I): 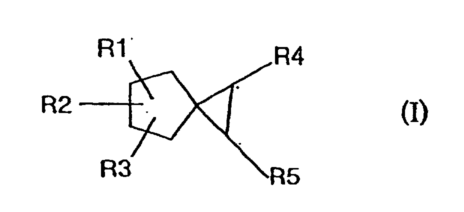 stereoisomers thereof, or pharmaceutically acceptable salts or hydrates thereof, wherein:
**R1** is (CH₂)ₙ(CH)ₘXY, where: n is 0-3, m is 0 or 1, X is CO₂H and Y is NH₂, with the proviso that, when m = 0, then n = 0 and the groups X and Y are directly attached to the ring,
**R2** and **R3** can be same or different and selected from the group comprising H, halo, alkyl, cycloalkyl, aryl or heterocycle, or when R2 and R3 are present on adjacent carbon atoms and taken together then R2 and R3 can form a cycloalkyl (3-6 carbon atoms), heterocycle, an aromatic ring or heteroaromatic ring,
R4 is selected from the group comprising H, halo, alkyl, cycloalkyl, aryl or heterocycle,
R5 is selected from the group comprising carboxyl, phosphono, phosphino, sulfono, sulfino, borono, tetrazol, isoxazol, -CH₂-carboxyl, -CH₂-phosphono, -CH₂-phosphino, -CH₂-sulfono, -CH₂-sulfino, -CH₂-borono, -CH₂-tetrazol, -CH₂-isoxazol, and higher homologues thereof.

2. A compound according to claim 1, wherein: **R1** is XY or CHXY.

3. A compound according to claim 1, wherein: **R5** is -CO₂H or -CH₂CO₂H.

4. A compound according to claim 1, wherein **R4** is H or Ph.

5. A compound according to claim 1, wherein: **R1** is (CH₂)ₙ(CH)ₘXY, where n = 0, m = 0, **R2** = **R3** = **R4** = H, **R5** is COOH.

6. A compound according to claim 1, wherein: **R1** is (CH₂)ₙ(CH)ₘXY, where n = 0, m = 0, **R2** = **R3** = H, **R4** is Ph and **R5** is COOH.

7. A compound according to claim 1, wherein: **R1** is (CH₂)ₙ(CH)ₘXY, where n = 0, m = 1, **R2** = **R3** = **R4** = H, **R5** is COOH.

8. A compound according to claim 1, wherein: **R1** is (CH₂)ₙ(CH)ₘXY, where n = 0, m = 0, **R2** and **R3** are present on adjacent carbon atoms and taken together form an aromatic ring, **R4** = H, **R5** is COOH.

9. The compound according to claim 1, wherein said compound is selected from the group:

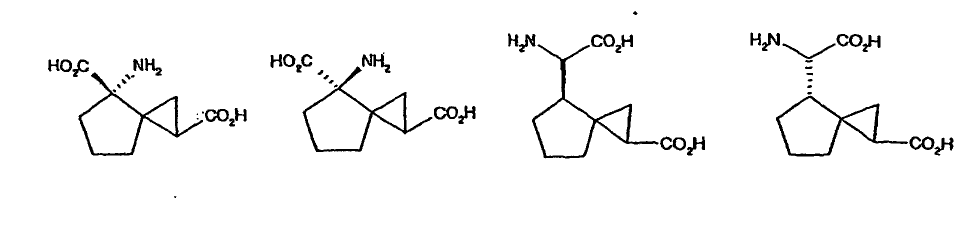

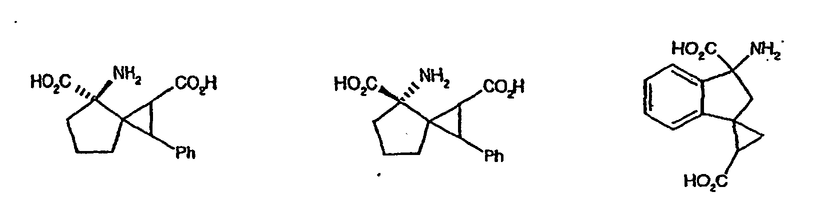

10. A process for the preparation of a compound of Formula I, 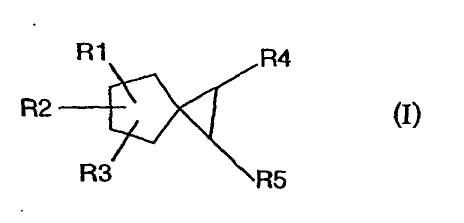 stereoisomers thereof, or pharmaceutically acceptable salts or hydrates thereof, wherein:
**R1** is (CH₂)ₙ(CH)ₘXY, where: n is 0-3, m is 0 or 1, X is CO₂H and Y is NH₂, with the proviso that, when m = 0, then n = 0 and the groups X and Y are directly attached to the ring,
**R2** and **R3** can be same or different and selected from the group comprising H, halo, alkyl, cycloalkyl, aryl or heterocycle, or when R2 and R3 are present on adjacent carbon atoms and taken together then R2 and R3 can form a cycloalkyl (3-6 carbon atoms), heterocycle, an aromatic ring or heteroaromatic ring,
R4 is selected from the group comprising H, halo, alkyl, cycloalkyl, aryl or heterocycle,
R5 is selected from the group comprising carboxyl, phosphono, phosphino, sulfono, sulfino, borono, tetrazol, isoxazol, -CH₂-carboxyl, -CH₂-phosphono, -CH₂-phosphino, -CH₂-sulfono, -CH₂-sulfino, -CH₂-borono, -CH₂-tetrazol, -CH₂-isoxazol, and higher homologues thereof, which comprises:
(a) hydrolyzing a compound of formula II: 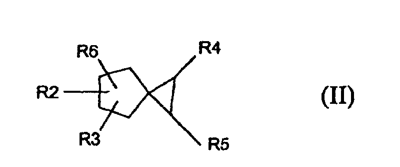 wherein: **R2, R3, R4, R5** are as defined above, and **R6** is: 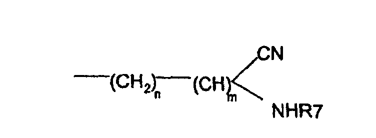 wherein:
n is 0-3, m is 0 or 1, with the proviso that, when m = 0, then n= 0 and the groups CN and NHR7 are directly attached to the ring, **R7** represents a hydrogen atom or an acyl group. Preferred values for **R7** are hydrogen and (C₁-C₂) alkanoyl groups, such as acetyl,
(b) hydrolyzing a compound of formula III: 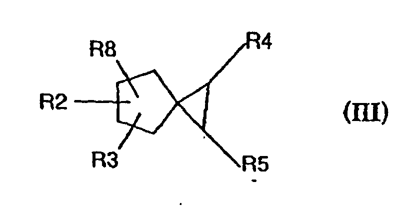 wherein: **R2, R3, R4, R5** are as defined above, and **R8** is: 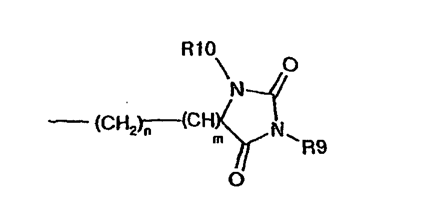 wherein: n is 0-3, m is 0 or 1, with the proviso that, when m = 0, then n= 0 and the cyclic group containing R9 and R10 is directly attached to the 5-membered ring, **R9** and **R10** each independently represent a hydrogen atom, a (C₂-C₆) alkanoyl group, a (C₁-C₄) alkyl group, a (C₂-C₄) alkenyl group or a phenyl (C₁-C₄) alkyl group in which the phenyl is unsubstituted or substituted by halogen, (C₁-C₄) alkyl or (C₁-C₄) alkoxy, or a salt thereof; or
(c) deprotecting a compound of formula IV: 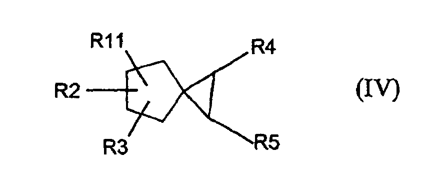 wherein: **R2, R3, R4, R5** are as defined above, and **R11** is: 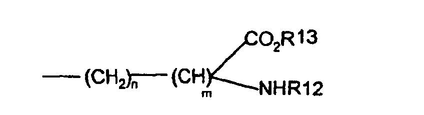 wherein:
n is 0-3, m is 0 or 1, with the proviso that, when m = 0, then n = 0 and the groups CO₂R13 and NHR12 are directly attached to the ring, **R13** represents a hydrogen atom or a carboxyl protecting group, or a salt thereof, and **R12** represents a hydrogen atom or a nitrogen protecting group; whereafter, if necessary and/or desired the following steps are carried out:
(i) resolving the compound of Formula I;
(ii) converting the compound of Formula I into a non-toxic metabolically-labile ester or amide thereof; and/or;
(iii) converting the compound of Formula I or a non-toxic metabolically-labile ester or amide thereof into a pharmaceutically acceptable salt thereof.

11. A pharmaceutical formulation, which comprises a compound as claimed in claim 1 and a pharmaceutically acceptable carrier, diluent or excipient.

12. A compound of structural formula (I) as claimed in claim 1, for use in modulating one or more metabotropic glutamate receptor functions in warm blooded mammals.

13. The use of the compound of structural formula (I) as claimed in claim 1, in the manufacture of a medicament for treating a neurological disease or disorder selected from the group comprising: cerebral deficits subsequent to cardiac bypass surgery and grafting, cerebral ischemia, stroke, cardiac arrest, spinal cord trauma, head trauma, perinatal hypoxia, and hypoglycemic neuronal damage, Alzheimer's disease, Huntington's Chorea, amyotrophic lateral sclerosis, AIDS-induced dementia, ocular damage, retinopathy, cognitive disorders, idiopathic and drug-induced Parkinson's disease, muscular spasms, convulsions, migraine headaches, urinary incontinence, psychosis, drug tolerance, withdrawal, and cessation (i.e. opiates, benzodiazepines, nicotine, cocaine, or ethanol), smoking cessation, anxiety and related disorders (e.g panic attack), emesis, brain edema, chronic pain, sleep disorders, Tourette's syndrome, attention deficit disorder, and tardive dyskinesia.

14. The use of the compound of structural formula (I), as claimed in claim 1, in the manufacture of a medicament for treating a psychiatric disease or disorder selected from the group comprising: schizophrenia, anxiety and related disorders (e.g. panic attack), depression, bipolar disorders, psychosis, and obsessive compulsive disorders.

15. A compound according to claim 12 or a use according to claim 13 or 14, wherein said compound is selected from the group of compounds comprising:

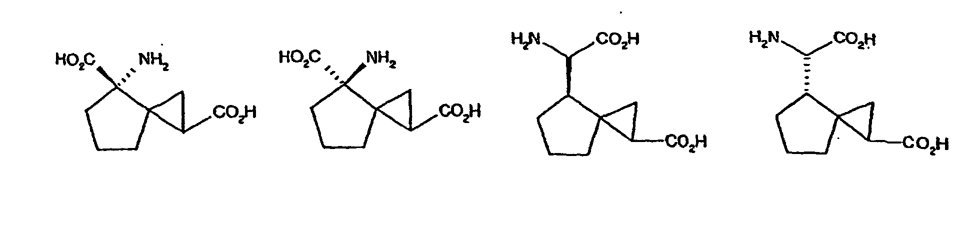

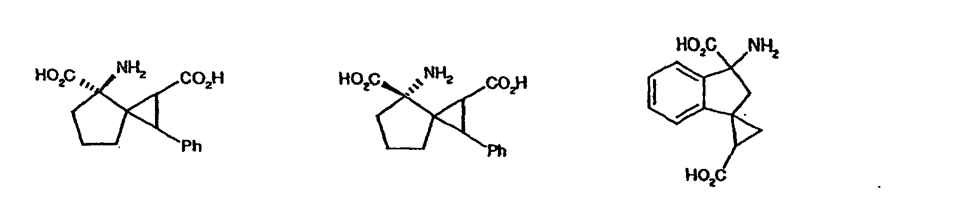

16. A compound of formula: 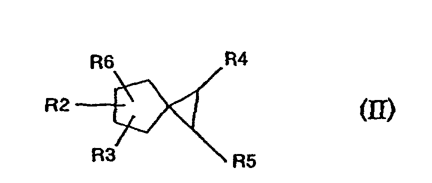 wherein: **R2, R3, R4, R5** are as defined above, and **R6** is: 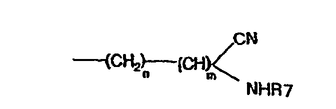 wherein:
n is 0-3, m is 0 or 1, with the proviso that, when m = 0, then n= 0 and the groups CN and NHR7 are directly attached to the ring, **R7** represents a hydrogen atom or an acyl group. Preferred values for **R7** are hydrogen and (C₁-C₂) alkanoyl groups, such as acetyl,

17. A compound of formula: 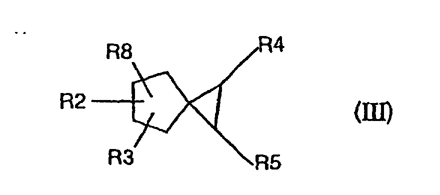 wherein: R2, R3, R4, R5 are as defined above, and R8 is: 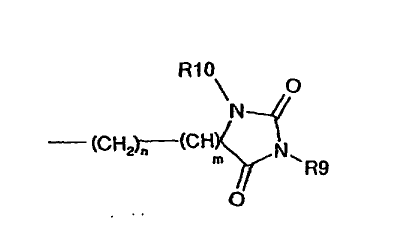 wherein: n is 0-3, m is 0 or 1, with the proviso that, when m = 0, then n= 0 and the cyclic group containing R9 and R10 is directly attached to the five membered ring, **R9** and **R10** each independently represent a hydrogen atom, a (C₂-C₆) alkanoyl group, a (C₁-C₄) alkyl group, a (C₂-C₄) alkenyl group or a phenyl (C₁-C₄) alkyl group in which the phenyl is unsubstituted or substituted by halogen, (C₁-C₄) alkyl or (C₁-C₄) alkoxy, or a salt thereof; or

18. A compound of formula: 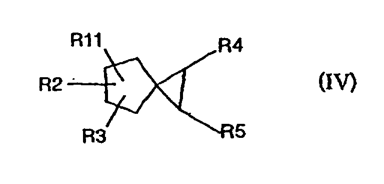 wherein: **R2, R3, R4, R5** are as defined above, and **R11** is: 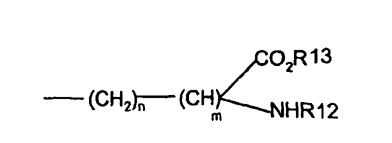 wherein:
n is 0-3, m is 0 or 1, with the proviso that, when m = 0, then n = 0 and the groups CO₂R13 and NHR12 are directly attached to the ring, **R13** represents a hydrogen atom or a carboxyl protecting group, or a salt thereof, and **R12** represents a hydrogen atom or a nitrogen protecting group.

## Patentansprüche

1. Verbindung mit der strukturellen Formel (I): 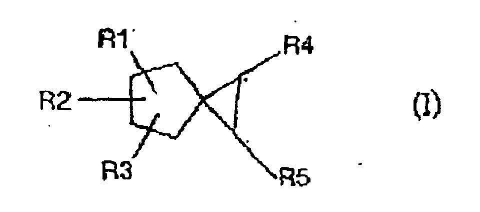 Stereoisomere davon oder pharmazeutisch akzeptable Salze oder Hydrat davon, wobei:
R1 gleich (CH₂)ₙ(CH)ₘXY ist, wobei n gleich 0-3, m gleich 0 oder 1, X gleich CO₂H und Y gleich NH₂ ist, unter der Voraussetzung, dass, wenn m = 0, dann n = 0 gilt und die Gruppen X und Y direkt an den Ring gebunden sind,
R2 und R3 identisch oder unterschiedlich sein und aus der Gruppe ausgewählt sein können, die H, Halo, Alkyl, Cycloalkyl, Aryl oder Heterocyclus enthält, oder - wenn R2 und R3 auf benachbarten Kohlenstoffatomen vorhanden sind und zusammengefasst werden - dann R2 und R3 ein Cycloalkyl (3-6 Kohlenstoffatome), einen Heterocyclus, einen aromatischen Ring oder einen heteroaromatischen Ring bilden können,
R4 aus der Gruppe ausgewählt ist, die H, Halo, Alkyl, Cycloalkyl, Aryl oder Heterocyclus aufweist,
R5 aus der Gruppe ausgewählt ist, die Carboxyl, Phosphono, Phosphino, Sulfono, Sulfino, Borono, Tetrazol, Isoxazol, -CH₂-Carboxyl, -CH₂-Phosphono, -CH₂-Phosphino, -CH₂-Sulfono, -CH₂-Sulfino, -CH₂-Borono, -CH₂-Tetrazol, -CH₂-Isoxazol und höhere Homologe davon enthält.

2. Verbindung nach Anspruch 1, wobei R1 gleich XY oder CHXY ist.

3. Verbindung nach Anspruch 1, wobei R5 gleich -CO₂H oder -CH₂CO₂H ist.

4. Verbindung nach Anspruch 1, wobei R4 gleich H oder Ph ist.

5. Verbindung nach Anspruch 1, wobei R1 gleich (CH₂)ₙ(CH)ₘXY ist, wobei gilt, dass n = 0, m = 0, R2 = R3 = R4 = H, R5 ist gleich COOH.

6. Verbindung nach Anspruch 1, wobei R1 gleich (CH₂)ₙ(CH)ₘXY ist, wobei gilt, dass n = 0, m = 0, R2 = R3 = H, R4 gleich Ph und R5 gleich COOH ist.

7. Verbindung nach Anspruch 1, wobei R1 gleich (CH₂)ₙ(CH)ₘXY ist, wobei gilt, dass n = 0, m = 1, R2 = R3 = R4 = H, R5 = COOH.

8. Verbindung nach Anspruch 1, wobei R1 gleich (CH₂)ₙ(CH)ₘXY ist, wobei gilt, dass n = 0, m = 0, R2 und R3 auf benachbarten Kohlenstoffatomen vorhanden sind und zusammengefasst einen aromatischen Ring bilden, R4 = H, R 5 gleich COOH ist

9. Verbindung nach Anspruch 1, wobei die Verbindung aus folgender Gruppe ausgewählt wird:

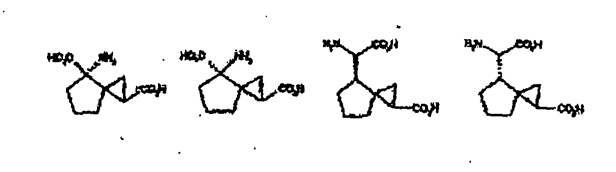

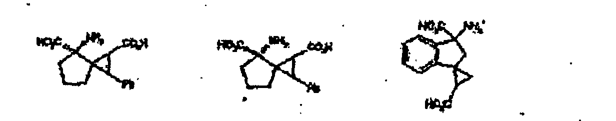

10. Verfahren zur Herstellung einer Verbindung gemäß Formel I, 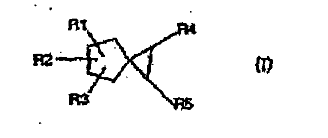 Stereoisomere davon oder pharmazeutisch akzeptable Salze oder Hydrate davon, wobei:
R1 gleich (CH₂)ₙ(CH)ₘXY ist, wobei n gleich 0-3, m gleich 0 oder 1, X gleich CO₂H und Y gleich NH₂ ist, unter der Voraussetzung, dass, wenn m = 0, dann n = 0 gilt und die Gruppen X und Y direkt an den Ring gebunden sind,
R2 und R3 identisch oder unterschiedlich sein und aus der Gruppe ausgewählt sein können, die H, Halo, Alkyl, Cycloalkyl, Aryl oder Heterocyclus enthält, oder - wenn R2 und R3 auf benachbarten Kohlenstoffatomen vorhanden sind und zusammengefasst werden - dann R2 und R3 ein Cycloalkyl (3-6 Kohlenstoffatome), einen Heterocyclus, einen aromatischen Ring oder einen heteroaromatischen Ring bilden können,
R4 aus der Gruppe ausgewählt ist, die H, Halo, Alkyl, Cycloalkyl, Aryl oder Heterocyclus aufweist.
R5 aus der Gruppe ausgewählt ist, die Carboxyl, Phosphono, Phosphino, Sulfono, Sulfino, Borono, Tetrazol, Isoxazol, -CH₂-Carboxyl, -CH₂-Phosphono, -CH₂-Phosphino, -CH₂-Sulfono, -CH₂-Sulfino, -CH₂-Borono, -CH₂-Tetrazol, -CH₂-Isoxazol und höhere Homologe davon enthält, wobei das Verfahren aufweist:
(a) Hydrolysieren einer Verbindung nach Formel II: 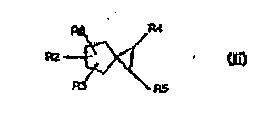 wobei R2, R3, R4, R5 obiger Definition entsprechen und R6 gleicb 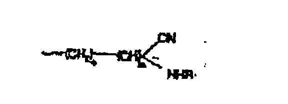 ist, wobei gilt, dass n gleich 0-3, m gleich 0 oder 1 ist, unter der Voraussetzung, dass, wenn m = 0, dann gilt, dass n = 0 und die Gruppen CN und NHR7 direkt an den Ring gebunden sind, R7 ein Wasserstoffatom oder eine Acylgruppe darstellt. Bevorzugte Werte für R7 sind Wasserstoff und (C₁-C₂)-Alkanoylgruppen wie Acetyl,
(b) Hydrolysieren einer Verbindung nach Formel III: 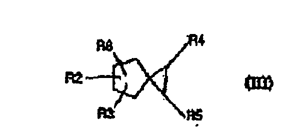 wobei R2, R3, R4, R5 obiger Definition entsprechen und R8 gleich 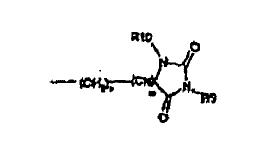 ist, wobei gilt, dass n gleich 0-3, m gleich 0 oder 1 ist, unter der Voraussetzung, dass, wenn m = 0, dann gilt, dass n = 0 und die cyclische Gruppe, welche R9 und R10 enthält, direkt an den fünfteiligen Ring gebunden ist, R9 und R10 jeweils unabhängig ein Wasserstoffatom, eine (C₂-C₆)Alkanoylgruppe, eine (C₁-C₄)Alkylgruppe, eine (C₂-C₄)Alkenylgruppe oder eine Phenyl(C₁-C₄)Alkylgruppe, in der das Phenyl unsubstituiert oder durch Halogen substituiert ist, (C₁-C₄)Alkyl oder (C₁-C₄)Alkoxy oder ein Salz davon darstellen; oder
(c) Entschützen einer Verbindung nach Formel IV: 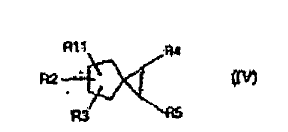 wobei R2, R3, R4, R5 obiger Definition entsprechen und R11 gleich 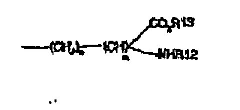 ist, wobei n gleich 0-3 ist, m gleich 0 oder 1 ist, unter der Voraussetzung, dass, wenn m = 0, dann gilt, dass n = 0 und die Gruppen CO₂R13 und NHR12 direkt an den Ring gebunden sind, R13 ein Wasserstoffatom oder eine Carboxylschutzgruppe oder ein Salz davon darstellt und R12 ein Wasserstoffatom oder eine Stickstoffschutzgruppe darstellt; wonach, falls erforderlich und/oder gewünscht, folgende Schritte ausgeführt werden:
(i) Auflösen der Verbindung gemäß Formel I,
(ii) Umwandeln der Verbindung gemäß Formel I in einen nicht-toxischen, metabolisch labilen Ester oder ein Amid davon; und/oder
(iii) Umwandeln der Verbindung gemäß Formel I oder eines nicht-toxischen, metabolisch labilen Esters oder Amids davon in ein pharmazeutisch akzeptables Salz davon.

11. Pharmazeutische Formulierung, welche eine Verbindung nach Anspruch 1 und einen pharmazeutisch akzeptablen Träger, ein pharmazeutisch akzeptables Verdünnungsmittel oder einen pharmazeutisch akzeptablen Arzneimittelträger aufweist.

12. Verbindung gemäß der Strukturformel (I) nach Anspruch 1 zur Verwendung beim Modulieren von einer oder mehreren Funktionen eines metabotropen Glutamatrezeptors in warmblütigen Säugetieren.

13. Verwendung der Verbindung gemäß der Strukturformel (I) nach Anspruch 1 in der Herstellung eines Medikamentes zur Behandlung einer neurologischen Erkrankung oder Störung, die aus der Gruppe ausgewählt ist, die zerebrale Defizite nach einer Herz-Bypass-Operation und Transplantation, zerebrale Iscbämie, Schlaganfall, Herzstillstand, Rückenmarksverletzung, Kopfverletzung, perinatale Hypoxie und hypoglykämischen neuronalen Schaden, Alzheimer-Krankheit, Huntington-Chorea, amyotrophe Lateralsklerose, AIDS-induzierte Dementia, Augenschäden, Retinopathie, kognitive Störungen, idiopathische und medikamenteninduzierte Parkinsonsche Krankheit, Muskelkrämpfe, Konvulsionen, Migräneschmerzen, Haminkontinenz, Psychose, Drogentoleranz, -entzug und - einstellung (z.B. Opiate, Benzodiazepine, Nikotin, Kokain oder Ethanol), Rauchentwöhnung, Angstzustände und damit verwandte Störungen (z.B. Panikattacken), Erbrechen, Himödem, chronische Schmerzen, Schlafstörungen, Tourette-Syndrom, Aufmerksamkeitsdefizitsyndrom und tardive Dyskinesia enthält.

14. Verwendung der Verbinthmg gemäß der Strukturformel (I) nach Anspruch 1 bei der Herstellung eines Medikamentes zur Behandlung einer psychiatrischen Erkrankung oder Störung, die aus der Gruppe ausgewählt ist, die Schizophrenie, Angstzustände und damit verwandte Störungen (z.B. Panikattacken), Depression, bipolare Störungen, Psychose und obsessiv-kompulsiven Störungen enthält.

15. Verbindung nach Anspruch 12 oder Verwendung nach Anspruch 13 oder 14, wobei die Verbindung aus der Gruppe von Verbindungen ausgewählt wird, die Folgendes umfasst:

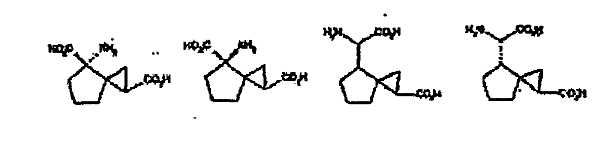

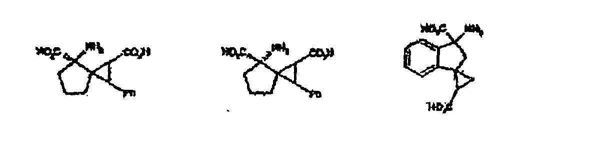

16. Verbindung der Formel: 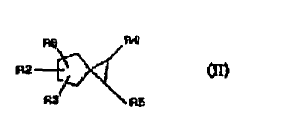 wobei R2, R3, R4, R5 obiger Definition entsprechen und R6 gleich 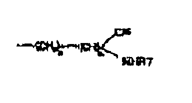 ist, wobei n gleich 0-3, m gleich 0 oder 1 ist, unter der Voraussetzung, dass, wenn m = 0, dann gilt, dass n = 0 und die Gruppen CN und NHR7 direkt an den Ring gebunden sind, R7 ein Wasserstoffatom oder eine Acylgruppe darstellt. Bevorzugte Werte für R7 sind Wasserstoff und (C₁-C₂)Alkanoylgruppen wie Acetyl.

17. Verbindung der Formel: 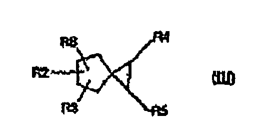 wobei R2, R3, R4, R5 obiger Definition entsprechen und R8 gleich 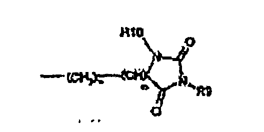 ist, wobei n gleich 0-3, m gleich 0 oder 1 ist, unter der Voraussetzung, dass, wenn m = 0, dann gilt, dass n = 0 und die cyclische Gruppe, welche R9 und R10 enthält, direkt an den fünfteiligen Ring gebunden ist, R9 und R10 jeweils unabhängig ein Wasserstoffatom, eine (C₂-C₆)Alkanoylgruppe, eine (C₁-C₄)Alkylgruppe, eine (C₂-C₄)Alkenylgruppe oder eine Phenyl(C₁-C₄)Alkylgruppe, in der das Phenyl unsubstituiert oder durch Halogen substituiert ist, (C₁-C₄)Alkyl oder (C₁-C₄)Alkoxy oder ein Salz davon darstellen; oder

18. Verbindung der Formel: 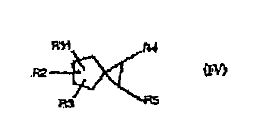 wobei R2, R3, R4, R5 obiger Definition entsprechen und R11 gleich 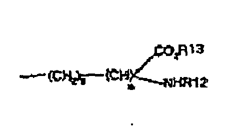 ist, wobei n gleich 0-3, m gleich 0 oder 1 ist, unter der Voraussetzung, dass, wenn m = 0, dann gilt, dass n = 0 und die Gruppen CO₂R13 und NHR12 direkt an den Ring gebunden sind, R13 ein Wasserstoffatom oder eine Carboxylschutzgruppe oder ein Salz davon darstellt, und R12 ein Wasserstoffatom oder eine Stickstoffschutzgruppe darstellt.

## Revendications

1. Composé ayant la formule structurelle (I) : 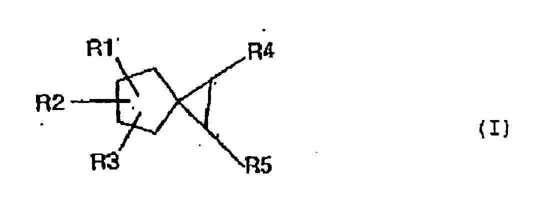 ses stéréoisomères ou ses sels ou hydrates pharmaceutiquement acceptables, formule dans laquelle :
R1 est (CH₂)ₙ(CH)ₘXY, où n est 0 à 3, m est 0 ou 1, X est CO₂H et Y est NH₂, à condition que, lorsque m=0, alors n=0 et les groupements X et Y soient directement fixés au cycle,
R2 et R3 peuvent être identiques ou différents et choisis dans le groupe constitué de H ou d'un groupement halo, alkyle, cycloalkyle, aryle ou hétérocycle, ou lorsque R2 et R3 sont présents sur des atomes de carbone adjacents et pris ensemble, alors R2 et R3 peuvent former un cycloalkyle (3 à 6 atomes de carbone), un hétérocycle, un cycle aromatique ou un cycle hétéroaromatique,
R4 est choisi dans le groupe constitué de H ou d'un groupement halo, alkyle, cycloalkyle, aryle ou hétérocycle,
R5 est choisi dans le groupe constitué d'un groupement carboxyle, phosphono, phosphino, sulfono, sulfino, borono, tétrazole, isoxazole, -CH₂-carboxyle, -CH₂-phosphono, -CH₂-phosphino, -CH₂-sulfono, -CH₂-sulfino, -CH₂-borono, -CH₂-tétrazole, -CH₂-isoxazole et leurs homologues supérieurs.

2. Composé selon la revendication 1, dans lequel R1 est XY ou CHXY.

3. Composé selon la revendication 1, dans lequel R5 est -CO₂H ou -CH₂CO₂H.

4. Composé selon la revendication 1, dans lequel R4 est H ou Ph.

5. Composé selon la revendication 1, dans lequel R1 est (CH₂)ₙ(CH)ₘXY, où n=0, m=0, R2=R3=R4=H, R5 est COOH.

6. Composé selon la revendication 1, dans lequel R1 est (CH₂)ₙ(CH)ₘXY, où n=0, m=0, R2=R3=H, R4 est Ph et R5 est COOH.

7. Composé selon la revendication 1, dans lequel R1 est (CH₂)ₙ(CH)ₘXY, où n=0, m=1, R2=R3=R4=H, R5 est COOH.

8. Composé selon la revendication 1, dans lequel R1 est (CH₂)ₙ(CH)ₘXY, où n=0, m=0, R2 et R3 sont présents sur des atomes de carbone adjacents et, pris ensemble, forment un cycle aromatique, R4=H, R5 est COOH.

9. Composé selon la revendication 1, dans lequel ledit composé est choisi dans le groupe constitué des suivants :

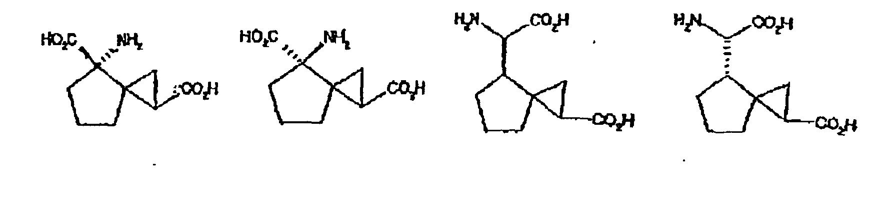

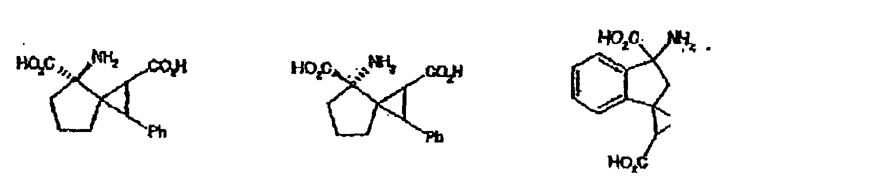

10. Procédé pour la préparation d'un composé de formule I : 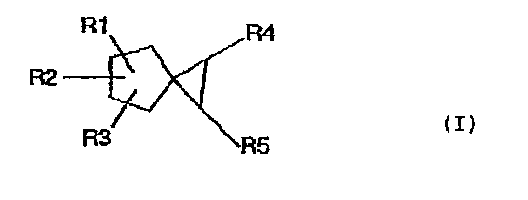 de ses stéréoisomères ou de ses sels ou hydrates pharmaceutiquement acceptables, dans lesquels :
R1 est (CH₂)ₙ(CH)ₘXY, où n est 0 à 3, m est 0 ou 1, X est CO₂H et Y est NH₂, à condition que, lorsque m=0, n=0 et les groupements X et Y soient directement fixés au cycle,
R2 et R3 peuvent être identiques ou différents et choisis dans le groupe constitué de H ou d'un groupement halo, alkyle, cycloalkyle, aryle ou hétérocycle ou, lorsque R2 et R3 sont présents sur des atomes de carbone adjacents et pris ensemble, alors R2 et R3 peuvent former un cycloalkyle (3 à 6 atomes de carbone), un hétérocycle, un cycle aromatique ou un cycle hétéroaromatique,
R4 est choisi dans le groupe constitué de H ou d'un groupement halo, alkyle, cycloalkyle, aryle ou hétérocycle,
R5 est choisi dans le groupe constitué des groupements carboxyle, phosphono, phosphino, sulfono, sulfino, borono, tétrazole, isoxazole, CH₂-carboxyle, -CH₂-phosphono, -CH₂-phosphino, -CH₂-sulfono, -CH₂-sulfino, -CH₂-borono, -CH₂-tétrazole, -CH₂-isoxazole et leurs homologues supérieurs, ledit procédé comprenant :
(a) l'hydrolyse d'un composé de formule II : 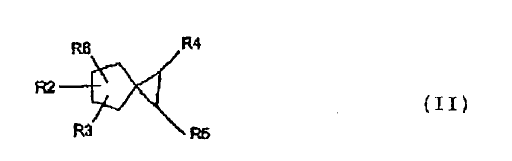 dans laquelle R2, R3, R4, R5 sont tels que définis ci-dessus et R6 a pour formule : 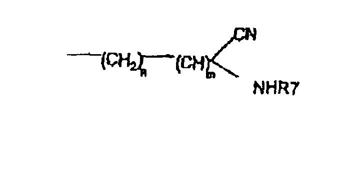 dans laquelle n est 0 à 3, m est 0 ou 1, à condition que, lorsque m=0, alors n=0 et les groupements CN et NHR7 soient directement fixés au cycle, R7 représente un atome d'hydrogène ou un groupement acyle, les valeurs préférées pour R7 étant l'hydrogène et les groupements alcanoyle en C₁-C₂ tels qu'un acétyle,
(b) l'hydrolyse d'un composé de formule III : 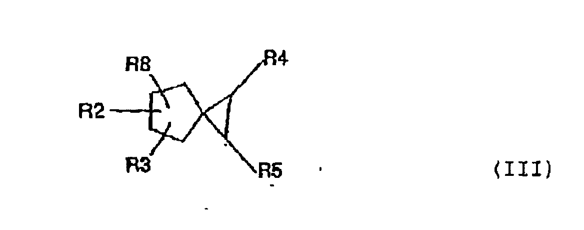 dans laquelle R2, R3, R4, R5 sont tels que définis ci-dessus et R8 a pour formule : 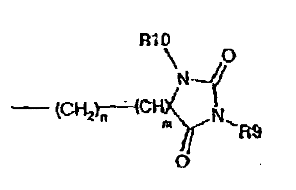 dans laquelle n est 0 à 3, m est 0 ou 1, à condition que, lorsque m=0, alors n=0 et le groupement cyclique contenant R9 et R10 soit directement fixé au cycle à 5 éléments, R9 et R10 représentent chacun indépendamment un atome d'hydrogène, un groupement alcanoyle en C₂-C₆, un groupement alkyle en C₁-C₄, un groupement alcényle en C₂-C₄ ou un groupement phénylalkyle(en C₁-C₄) dans lequel le phényle est non substitué ou est substitué par un halogène, un alkyle en C₁-C₄ ou un alcoxy en C₁-C₄ ou un de leurs sels; ou
(c) la déprotection d'un composé de formule IV : 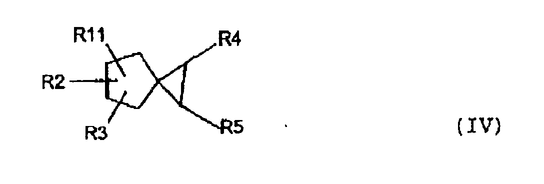 dans laquelle R2, R3, R4, R5 sont tels que définis ci-dessus et R11 a pour formule : 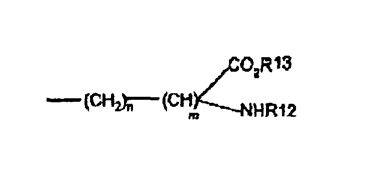 dans laquelle n est 0 à 3, m est 0 ou 1, à condition que, lorsque m=0, alors n=0 et les groupes CO₂R13 et NHR12 soient directement fixés au cycle, R13 représente un atome d'hydrogène ou un groupement de protection carboxyle, ou un de ses sels, et R12 représente un atome d'hydrogène ou un groupement de protection azote; après quoi, si nécessaire et/ou souhaité, les étapes suivantes sont réalisées :
(i) résolution du composé de formule I;
(ii) conversion du composé de formule I en un de ses esters ou amides non toxiques métaboliquement labiles; et/ou
(iii) conversion du composé de formule I ou un de ses esters ou amides non toxiques métaboliquement labiles en un de leurs sels pharmaceutiquement acceptables.

11. Formulation pharmaceutique, qui comprend un composé selon la revendication 1 et un véhicule, un diluant ou un excipient pharmaceutiquement acceptable.

12. Composé de formule structurelle (I) selon la revendication 1 pour usage pour moduler une ou plusieurs fonctions réceptrices de glutamate métabotropes chez les mammifères à sang chaud.

13. Utilisation du composé de formule structurelle (I) selon la revendication 1 dans la fabrication d'un médicament pour traiter une maladie ou un trouble neurologique choisi(e) dans le groupe comprenant des déficits cérébraux à la suite d'une opération de pontage cardiaque et de greffe cardiaque, une ischémie cérébrale, une attaque, un arrêt cardiaque, un traumatisme de la moelle épinière, un traumatisme crânien, une hypoxie périnatale, une lésion neuronale hypoglycémique, la maladie d'Alzheimer, la chorée de Huntington, la sclérose latérale amyotrophique, la démence induite par le SIDA, une lésion oculaire, la rétinopathie, les troubles cognitifs, la maladie de Parkinson idiopathique et d'origine médicamenteuse, les spasmes musculaires, les convulsions, les maux de tête migraineux, l'incontinence urinaire, la psychose, la tolérance médicamenteuse, le sevrage et l'arrêt (c'est-à-dire de l'usage des opiacées, des benzodiazépines, de la nicotine, de la cocaïne ou de l'alcool), la désaccoutumance du tabac, l'anxiété et les troubles apparentés (par exemple la crise de panique), les vomissements, l'oedème cérébral, la douleur chronique, les troubles du sommeil, le syndrome de Tourette, le trouble de déficit de l'attention et la dyskinésie tardive.

14. Utilisation du composé de formule structurelle (I) selon la revendication 1 dans la fabrication d'un médicament pour traiter une maladie ou un trouble psychiatrique choisi(e) dans le groupe comprenant la schizophrénie, l'anxiété et les troubles apparentés (par exemple la crise de panique), la dépression, les troubles bipolaires, la psychose et les troubles obsessionnels compulsifs.

15. Composé selon la revendication 12 ou utilisation selon la revendication 13 ou 14, dans lequel ledit composé est choisi dans le groupe constitué des composés comprenant :

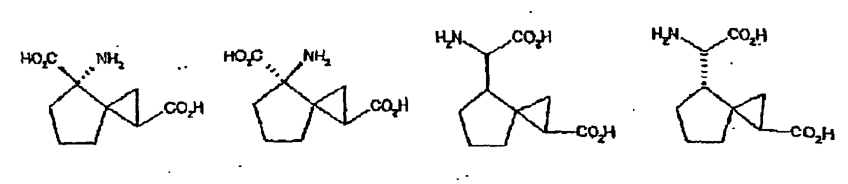

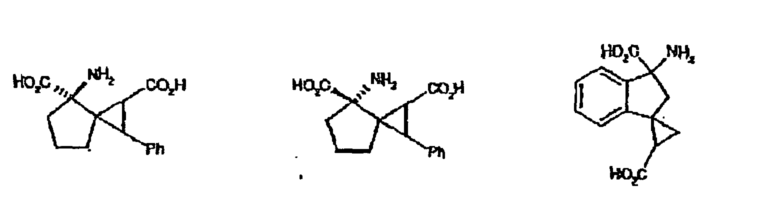

16. Composé de formule : 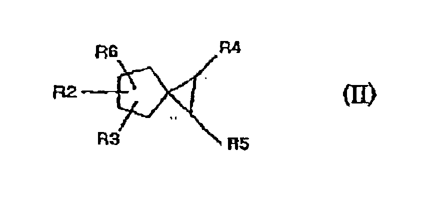 dans laquelle R2, R3, R4, R5 sont tels que définis ci-dessus et R6 a pour formule : 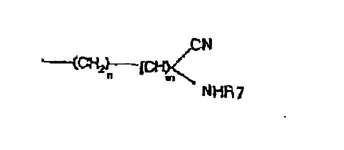 dans laquelle n est 0 à 3, m est 0 ou 1, à condition que lorsque m=0, alors n=0 et les groupements CN et NHR7 soient directement fixés au cycle, R7 représente un atome d'hydrogène ou un groupement acyle. Les valeurs préférées de R7 sont l'hydrogène et les groupements alcanoyle en C₁-C₂, tels que l'acétyle.

17. Composé de formule : 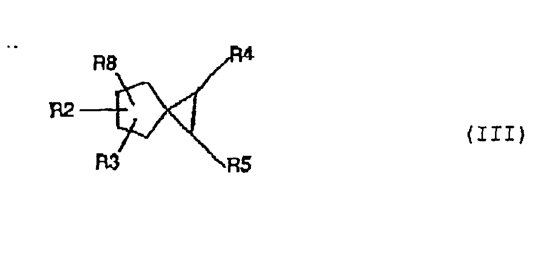 dans laquelle R2, R3, R4, R5 sont tels que définis ci-dessus et R8 a pour formule : 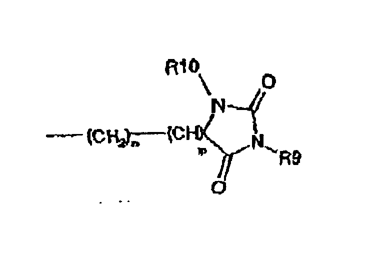 dans laquelle n est 0 à 3, m est 0 ou 1, à condition que, lorsque m=0, alors n=0 et que le groupement cyclique contenant R9 et R10 soit directement fixé au cycle à cinq chaînons, R9 et R10 représentent chacun indépendamment un atome d'hydrogène, un groupement alcanoyle en C₂-C₆, un groupement alkyle en C₁-C₄, un groupement alcényle en C₂-C₄ ou un groupement phényl alkyle (en C₁-C₄) dans lequel le phényle est non substitué ou est substitué par un halogène, un alkyle en C₁-C₄ ou un alcoxy en C₁-C₄ ou un de leurs sels.

18. Composé de formule : 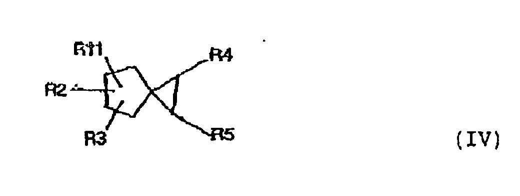 dans laquelle R2, R3, R4, R5 sont tels que définis ci-dessus et R11 a pour formule : 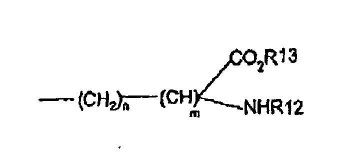 dans laquelle n est 0 à 3, m est 0 ou 1, à condition que, lorsque m=0, alors n=0 et les groupements CO₂R13 et NHR12 soient directement fixés au cycle, R13 représente un atome d'hydrogène ou un groupement de protection carboxyle ou un de ses sels et R12 représente un atome d'hydrogène ou un groupement de protection azote.
